(19) Europäisches Patentamt
European Patent Office
Office européen des brevets

(11) **EP 4 056 110 B1**

(12) **EUROPEAN PATENT SPECIFICATION**

(45) Date of publication and mention
of the grant of the patent:
**09.10.2024 Bulletin 2024/41**

(21) Application number: **19952100.6**

(22) Date of filing: **08.11.2019**

(51) International Patent Classification (IPC):
*A61B 5/00* (2006.01)        *A61B 5/02* (2006.01)
*A61B 5/0275* (2006.01)      *A61B 5/107* (2006.01)
*A61B 34/10* (2016.01)       *G06T 7/00* (2017.01)
*G06T 7/11* (2017.01)

(52) Cooperative Patent Classification (CPC):
**G06T 7/0016; A61B 5/0033; A61B 5/02028;
A61B 5/0275; A61B 5/1072; A61B 5/489;
G06T 7/11;** A61B 2034/105; A61B 2576/00;
G06T 2207/30104; G06T 2207/30172

(86) International application number:
**PCT/CN2019/116674**

(87) International publication number:
**WO 2021/087968 (14.05.2021 Gazette 2021/19)**

(54) **METHOD AND APPARATUS FOR CALCULATING AN ADJUSTED BLOOD FLOW VELOCITY IN LARGEST CONGESTION STATE ON BASIS OF MICROCIRCULATION RESISTANCE INDEX**

VERFAHREN UND VORRICHTUNG ZUR BERECHNUNG EINER ANGEPASSTEN BLUTSTRÖMUNGSGESCHWINDIGKEIT IM GRÖSSTEN ÜBERLASTUNGSZUSTAND AUF DER BASIS EINES MIKROZIRKULATIONSWIDERSTANDSINDEX

PROCÉDÉ ET APPAREIL DE CALCUL D'UNE VITESSE D'ÉCOULEMENT SANGUIN AJUSTÉE DANS L'ÉTAT DE CONGESTION LE PLUS GRAND SUR LA BASE D'UN INDICE DE RÉSISTANCE DE MICROCIRCULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.11.2019 CN 201911065694**

(43) Date of publication of application:
**14.09.2022 Bulletin 2022/37**

(73) Proprietor: **Suzhou Rainmed Medical Technology
Co., Ltd.
Suzhou, Jiangsu 215000 (CN)**

(72) Inventors:
• **LIU, Guangzhi
Suzhou, Jiangsu 215000 (CN)**
• **GONG, Yanjun
Suzhou, Jiangsu 215000 (CN)**
• **LI, Jianping
Suzhou, Jiangsu 215000 (CN)**

• **YI, Tieci
Suzhou, Jiangsu 215000 (CN)**
• **ZHENG, Bo
Suzhou, Jiangsu 215000 (CN)**

(74) Representative: **Boult Wade Tennant LLP
Salisbury Square House
8 Salisbury Square
London EC4Y 8AP (GB)**

(56) References cited:
CN-A- 1 559 345      CN-A- 107 730 540
CN-A- 108 305 246    CN-A- 108 550 189
CN-A- 108 742 570    CN-A- 110 024 041
CN-A- 110 384 494    US-A1- 2019 029 519

• **No further relevant documents disclosed**

EP 4 056 110 B1

**Description**

TECHNICAL FIELD

The present disclosure relates to the field of coronary artery technology, and in particular, to a method and an apparatus for calculating an adjusted blood flow velocity in maximum hyperemia state based on index for microcirculatory resistance, a coronary artery analysis system and a computer storage medium.

BACKGROUND

According to the statistics of the World Health Organization, cardiovascular diseases have become a "leading killer" of human health. In recent years, the analysis of the physiological and pathological behaviors of cardiovascular diseases using hemodynamics has also become a very important means of diagnosis of the cardiovascular diseases.

Blood flow quantity and flow velocity are very important parameters of hemodynamics. How to measure the blood flow quantity and flow velocity accurately and conveniently has become the focus of many researchers.

Due to different vital signs of different populations, evaluation standards for normal values are slightly different. For example, the myocardial microcirculation function of the elderly is relatively poor, and the blood flow velocity is generally lower than that of the young. If the industry general evaluation standard is used, the blood flow velocity used will be higher than the actual value. The higher blood flow velocity will further affect the coronary artery evaluation parameters, such as: fractional flow reserve FFR, fractional flow reserve during a diastolic phase iFR, and index for microcirculatory resistance iFMR during the diastolic phase.

Therefore, at this stage, how to obtain the index for microcirculatory resistance iFMR according to individual differences, and then calculate an adjusted blood flow velocity in the maximum hyperemia state according to the index for microcirculatory resistance iFMR, to obtain a more targeted blood flow velocity with individualized differences and improve the accuracy of the blood flow velocity calculation, has become an urgent problem in the field of coronary artery technology.

CN 110 384 494 A and CN 108 550 189 A disclose devices and methods for calculating the blood flow velocity.

SUMMARY

The present disclosure provides a method and an apparatus for calculating an adjusted blood flow velocity in maximum hyperemia state based on index for microcirculatory resistance, a coronary artery analysis system and a computer storage medium, so as to solve the problem of how to obtain a more targeted, individualized blood flow velocity in the maximum hyperemia state according to individualized differences.

In order to achieve the above object, in a first aspect, the present disclosure provides a method for calculating an adjusted blood flow velocity in maximum hyperemia state based on index for microcirculatory resistance, comprising:

acquiring an index for microcirculatory resistance iFMR during a diastolic phase according to a blood flow velocity v, an aortic pressure waveform, and an physiological parameter;
making an adjustment parameter r equal to 1 if the index for microcirculatory resistance iFMR during the diastolic phase is less than K;
making the adjustment parameter r satisfy a formula

$$r = 1 - \frac{\text{iFMR} - K}{100}$$

if the index for microcirculatory resistance iFMR during the diastolic phase is greater than or equal to K, wherein K is a positive number less than 100;
acquiring a corrected blood flow velocity in a maximum hyperemia state according to a formula $v' = rv_h$;
wherein v' represents the corrected blood flow velocity in the maximum hyperemia state, and $v_h$ represents a blood flow velocity in the maximum hyperemia state.

Optionally, in the above method for calculating an adjusted blood flow velocity in maximum hyperemia state based on index for microcirculatory resistance,

$$v_h = z\bar{v} + x$$

wherein $v_h$ represents the blood flow velocity in the maximum hyperemia state, v represents an average blood flow velocity in a heartbeat cycle area, z is a constant in the range of 1 to 3, and x is a constant in the range of 50 to 300; K=50.

Optionally, in the above method for calculating an adjusted blood flow velocity in maximum hyperemia state based on index of microcirculatory resistance, a manner for acquiring an index for microcirculatory resistance iFMR during a diastolic phase according to a blood flow velocity v, an aortic pressure waveform, and an physiological parameter comprises:

selecting a maximum value of the blood flow velocity v, i.e., a maximum blood flow velocity $v_{max}$ during the diastolic phase;
a time period corresponding to the $v_{max}$ being the diastolic phase, acquiring an average aortic pressure during the diastolic phase according to the aortic pressure waveform;

$$\mathrm{iFMR} = \overline{P_a}\big/v_{\max} \times k + c \; ;$$

$$\overline{P_a} = \frac{\sum_1^j \left(P_{a1} + P_{a2} \dots P_{aj}\right)}{j} \; ;$$

wherein, $\overline{P_a}$ represents the average aortic pressure during the diastolic phase; $P_{a1}$, $P_{a2}$, and $P_{aj}$ represent aortic pressures corresponding to a first point, a second point, and a j-th point within the diastolic phase on the aortic pressure waveform, respectively, and j represents the number of pressure points contained in the aortic pressure waveform during the diastolic phase, $v_h$ represents the blood flow velocity in the maximum hyperemia state obtained by selecting a maximum value from all blood flow velocities v; k and c represent the influence parameters k=1~3, c=0~10.

Optionally, in the above method for calculating an adjusted blood flow velocity in maximum hyperemia state based on index for microcirculatory resistance, the influence parameter k=a×b, wherein a represents a characteristic value of diabetes, b represents a characteristic value of hypertension, and c represents gender.

Optionally, in the above method for calculating an adjusted blood flow velocity in maximum hyperemia state based on index for microcirculatory resistance, if a patient does not suffer from diabetes, then 0.5≤a≤1; if the patient suffers from diabetes, then 1 < a≤2;

if the patient's blood pressure is greater than or equal to 90 mmHg, then 1<b≤1.5; if the patient's blood pressure is less than 90 mmHg, then 0.5≤ b≤1;
if the patient is male, then c=0; if the patient is female, then c=3~10.

Optionally, in the above method for calculating an adjusted blood flow velocity in maximum hyperemia state based on index for microcirculatory resistance, if the patient does not suffer from diabetes, then a=1; If the patient suffers from diabetes, then a=2;

if the patient's blood pressure is greater than or equal to 90 mmHg, then b=1.5; if the patient's blood pressure is less than 90 mmHg, then b=1;
if the patient is male, c=0; if the patient is female, c=5.

Optionally, in the above method for calculating an adjusted blood flow velocity in maximum hyperemia state based on index for microcirculatory resistance, a manner for acquiring a blood flow velocity comprises:

reading a group of two-dimensional coronary artery angiogram images of at least one body position;
extracting a blood vessel segment of interest from the group of two-dimensional coronary artery angiogram images;
extracting a centerline of the blood vessel segment;
determining a difference in time taken for a contrast agent flowing through the blood vessel segment in any two frames of the two-dimensional coronary artery angiogram images with the difference being Δt, and determining a difference in centerline length of a sub-segment of the blood vessel segment through which the contrast agent flows in the two frames of two-dimensional coronary artery angiogram image with the difference being ΔL;
solving the blood flow velocity according to a ratio of ΔL to Δt.

Optionally, in the above method for calculating an adjusted blood flow velocity in maximum hyperemia state based on index for microcirculatory resistance, a manner for extracting a blood vessel segment of interest from the group of two-dimensional coronary artery angiogram images comprises:

selecting N frames of the two-dimensional coronary artery angiogram images from the group of two-dimensional coronary artery angiogram images;
acquiring the blood vessel segment of interest by picking a beginning point and an ending point of the blood vessel of interest on the two-dimensional coronary artery angiogram images.

Optionally, in the above method for calculating an adjusted blood flow velocity in maximum hyperemia state based on index for microcirculatory resistance, a manner for extracting the centerline of the blood vessel segment comprises:

extracting a blood vessel skeleton from the two-dimensional coronary artery angiogram images;
according to the extension direction of the blood vessel segment and the principle of obtaining the shortest path between two points;
extracting the centerline of the blood vessel segment along the blood vessel skeleton.

Optionally, in the above method for calculating an adjusted blood flow velocity in maximum hyperemia state based on index for microcirculatory resistance, a manner for determining a difference in time taken for a contrast agent flowing through the blood vessel segment in any two frames of the two-dimensional coronary artery angiogram images with the difference being Δt, and determining a difference in centerline length of a sub-segment of the blood vessel segment through which the contrast agent flows in the two frames of two-dimensional coronary artery angiogram image with the difference be-

ing $\Delta L$, and solving the blood flow velocity according to the ratio of $\Delta L$ to $\Delta t$ comprises:

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;

solving the time difference and centerline length difference of the N-th frame of image and a (N-1)th frame, ..., a (N-b)th frame, ..., a (N-a)th frame, ..., the first frame of image, successively, with the time differences being $\Delta t_1$,..., $\Delta t_b$,..., $\Delta t_a$,..., $\Delta t_{N-1}$, respectively; the centerline length differences being $\Delta L_1$,..., $\Delta L_b$,..., $\Delta L_a$,..., $\Delta L_{N-1}$, respectively;

$$v = \frac{\Delta L}{\Delta t}$$

according to , obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1$,..., $v_b$,..., $v_a$,..., $v_{N-1}$, respectively.

Optionally, in the above method for calculating an adjusted blood flow velocity in maximum hyperemia state based on index for microcirculatory resistance, a manner for determining a difference in time taken a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$ , and solving the blood flow velocity according to the ratio of $\Delta L$ to $\Delta t$ comprises:

solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image, successively;

$$v = \frac{\Delta L}{\Delta t}$$

according to , obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

Optionally, in the above method for calculating an adjusted blood flow velocity in maximum hyperemia state based on index for microcirculatory resistance, after the manner for extracting a centerline of the blood vessel segment, and before the manner for determining a difference in time taken for a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$ , the method further comprises:

reading a group of two-dimensional coronary artery angiogram images of at least two body positions;

acquiring geometric structure information of the blood vessel segment;

performing graphics processing on the blood vessel segment of interest;

extracting a blood vessel contour line of the blood vessel segment;

according to the geometric structure information of the blood vessel segment, synthesizing a three-dimensional blood vessel model by projecting the at least two body positions' two-dimensional coronary angiogram images which have been extracted centerline and contour line of the blood vessel onto a three-dimensional plane.

Optionally, in the above method for calculating an adjusted blood flow velocity in maximum hyperemia state based on index for microcirculatory resistance, a manner for solving the blood flow velocity according to the ratio of $\Delta L$ to $\Delta t$ comprises:

according to the three-dimensional blood vessel model, acquiring a centerline of the three-dimensional blood vessel model, correcting the centerline extracted from the two-dimensional coronary angiogram images, and correcting the centerline difference $\Delta L$ to obtain $\Delta L$';

solving the blood flow velocity v according to the ratio of the $\Delta L$' to the $\Delta t$.

In a third aspect, the present disclosure provides an apparatus for calculating an adjusted blood flow velocity in maximum hyperemia state based on index for microcirculatory resistance, used for the above method for adjusting blood flow velocity in maximum hyperemia state based on index for microcirculatory resistance, comprising: a blood flow velocity acquisition unit , an aortic pressure waveform acquisition unit, a physiological parameter acquisition unit, an unit of index for microcirculatory resistance during diastolic phase and an adjustment parameter unit; the unit of index for microcirculatory resistance during diastolic phase is connected with the blood flow velocity acquisition unit, the aortic pressure waveform acquisition unit and the physiological parameter acquisition unit;

the blood flow velocity acquisition unit is configured to acquire a blood flow velocity v;

the aortic pressure waveform acquisition unit is configured to acquire, in real time, an aortic pressure waveform changing over time;

the physiological parameter acquisition unit is configured to acquire physiological parameters of a patient, comprising gender and disease history;

the unit of index for microcirculatory resistance during diastolic phase is configured to receive the blood flow velocity v, the aortic pressure waveform, and the physiological parameters sent by the blood flow velocity acquisition unit, the aortic pressure waveform acquisition unit, and the physiological parameter acquisition unit, and then to obtain an index for microcirculatory resistance iFMR during a diastolic phase according to the blood flow velocity v, the aortic pressure waveform, and the physiological parameters;

the adjustment parameter unit is configured to receive iFMR value of the unit of index for microcirculatory resistance during diastolic phase; make an adjustment parameter r equal to1 if the index for microcirculatory resistance during the diastolic phase iFMR<K; make the adjustment parameter r to satisfy

$$r = 1 - \frac{\text{iFMR} - K}{100}$$

a formula                    if the index for microcirculatory resistance during the diastolic phase iFMR≥K; where K is a positive number less than 100.

Optionally, the above apparatus for calculating an adjusted blood flow velocity in maximum hyperemia state based on index for microcirculatory resistance further comprises: an image reading unit, a blood vessel segment extraction unit, and a centerline extraction unit connected in sequence, a time difference unit and the physiological parameter acquisition unit both connected to the image reading unit, and the blood flow velocity acquisition unit that respectively connected with the time difference unit and a centerline difference unit, respectively; the centerline difference unit is connected with the centerline extraction unit;

the image reading unit is configured to read a group of two-dimensional coronary artery angiogram image of at least one body position;

the blood vessel segment extraction unit is configured to receive two-dimensional coronary artery angiogram images sent by the image reading unit, and to extract a blood vessel segment of interest in the images;

the centerline extraction unit is configured to receive the blood vessel segment sent by the blood vessel segment extraction unit, and to extract the centerline of the blood vessel segment;

the time difference unit is configured to receive any two frames of the two-dimensional coronary artery angiogram images sent by the image reading unit, and to determine a difference in time taken for a contrast agent flowing through the blood vessel segment in the two frames of two-dimensional coronary artery angiogram image with the difference being $\Delta t$;

the centerline difference unit is configured to receive the centerline of a sub-segment of the blood vessel segment flowed through by the contrast agent in the two frames of two-dimensional coronary artery angiogram image sent by the centerline extraction unit, and to determine a difference in centerline length of the sub-segment of the blood vessel segment through which the contrast agent flows in the two frames of two-dimensional coronary artery angiogram image with the difference being $\Delta L$ ;

the blood flow velocity acquisition unit comprises a blood flow velocity calculation module and a diastolic blood flow velocity calculation module, the blood flow velocity calculation module being respectively connected to the time difference unit and the centerline difference unit, the diastolic blood flow velocity calculation module being connected with the blood flow velocity calculation module;

the blood flow velocity calculation module is configured to receive the $\Delta L$ and the $\Delta t$ sent by the time difference unit and the centerline difference unit, and to solve the blood flow velocity according to the ratio of $\Delta L$ to $\Delta t$;

the diastolic blood flow velocity calculation module is configured to receive the blood flow velocity sent by the blood flow velocity calculation module, and to select a maximum value of the blood flow velocity as a blood flow velocity during a diastolic phase;

the physiological parameter acquisition unit is configured to receive the two-dimensional coronary artery angiogram images of the image reading unit, to acquire a physiological parameter of a patient and image shooting angles, and to transmit the physiological parameter and image shooting angles to the unit of index for microcirculatory resistance during diastolic phase.

Optionally, the above apparatus for calculating an adjusted blood flow velocity in maximum hyperemia state based on index of microcirculatory resistance further comprises: a blood vessel skeleton extraction unit and a three-dimensional blood vessel reconstruction unit, both connected to the image reading unit, a contour line extraction unit connected to the blood vessel skeleton extraction unit, the three-dimensional blood vessel reconstruction unit being connected with the physiological parameter acquisition unit, the centerline extraction unit and the contour line extraction unit;

the blood vessel skeleton extraction unit is configured to receive the two-dimensional coronary artery angiogram images sent by the image reading unit, and to extract a blood vessel skeleton in the images;

the contour line extraction unit is configured to receive the blood vessel skeleton of the blood vessel skeleton extraction unit, and to extract a contour line of the blood vessel segment of interest according to the blood vessel skeleton;

the three-dimensional blood vessel reconstruction unit is configured to receive the contour line, the image shooting angles and the centerline sent by the contour line extraction unit, the physiological parameter acquisition unit and the centerline extraction unit, and to receive the two-dimensional coronary artery angiogram images sent by the image reading unit in order to synthesize a three-dimensional blood vessel model by projecting at least two body positions' two-dimensional coronary angiogram images which have been extracted centerline and contour line of the blood vessel onto a three-dimensional plane according to the geometric structure information of the blood vessel segment;

the centerline extraction unit is configured to re-extract the centerline of the blood vessel segment from the three-dimensional blood vessel model of the three-dimensional blood vessel reconstruction unit, and to re-acquire the length of the centerline.

In a fourth aspect, the present disclosure provides a coronary artery analysis system, comprising: the apparatus for calculating an adjusted blood flow velocity in maximum hyperemia state based on index for microcirculatory resistance according to any one of the above.

In a fifth aspect, the present disclosure provides a computer storage medium having stored thereon a computer program to be executed by a processor, the above method for calculating an adjusted blood flow velocity in maximum hyperemia state based on index for microcirculatory resistance is implemented when the computer program is executed by the processor.

The beneficial effects brought about by the solutions provided by the embodiments of the present disclosure comprise at least the following:

According to the present disclosure, an index for microcirculatory resistance iFMR during a diastolic phase is acquired according to a blood flow velocity v, an aortic pressure waveform, and an physiological parameter; then an adjustment parameter is obtained by comparing iFMR with K, where the adjustment parameter varies for different values of iFMR, further, a differentiated parameter is obtained according to individualized differences, placing a solid foundation for the accuracy of a blood vessel calculation parameter, then, a corrected blood flow velocity in the maximum hyperemia state is obtained by a product of the adjustment parameter and the blood flow velocity in the maximum hyperemia state, allowing for more targeted, individualized and more accurate measurement results.

BRIEF DESCRIPTION OF DRAWINGS

The drawings illustrated here are used to provide a further understanding of the present disclosure and constitute a part of the present disclosure. The exemplary embodiments and the descriptions thereof are used to explain the present disclosure, and do not constitute an improper limitation on the present disclosure. In the drawings:

FIG. 1 is a flowchart of a method for calculating an adjusted blood flow velocity in maximum hyperemia state based on index for microcirculatory resistance of the present disclosure;
FIG. 2 is a flowchart of S100 of the present disclosure;
FIG. 3 is a flowchart of S120 of the present disclosure;
FIG. 4 is a flowchart of S130 of the present disclosure;
FIG. 5 is a flowchart of an embodiment of S140 of the present disclosure;
FIG. 6 is a flowchart of another embodiment of S140 of the present disclosure;
FIG. 7 is a structural block diagram of an embodiment of an apparatus for calculating an adjusted blood flow velocity in maximum hyperemia state based on index for microcirculatory resistance of the present disclosure;
FIG. 8 is a structural block diagram of another embodiment of an apparatus for calculating an adjusted blood flow velocity in maximum hyperemia state based on index for microcirculatory resistance of the present disclosure;

Reference numerals are explained below:
blood flow velocity acquisition unit 1, blood flow velocity calculation module 101, diastolic blood flow velocity calculation module 102, aortic pressure waveform acquisition unit 2, physiological parameter acquisition unit 3, unit of index for microcirculatory resistance during diastolic phase 4, adjustment parameter unit 5, image reading unit 6, blood vessel segment extraction unit 7, centerline extraction unit 8, time difference unit 9, centerline difference unit 10, blood vessel skeleton extraction unit 11, three-dimensional blood vessel reconstruction unit 12, contour line extraction unit 13, flow velocity correction unit 14, unit of flow velocity in maximum hyperemia state 15.

DETAILED DESCRIPTION OF PREFERRED EMBODIMENTS

In order to make objects, technical solutions and advantages of the present disclosure clearer, the technical solutions of the present disclosure will be clearly and completely described below with reference to the specific embodiments and corresponding drawings. It is apparent that the described embodiments are merely part of the embodiments of the present disclosure rather than all of them. Based on the embodiments in the present disclosure, without making creative work, all the other embodiments obtained by a person skilled in the art will fall into the protection scope of the present disclosure.

Hereinafter, a number of embodiments of the present disclosure will be disclosed with drawings. For clear illustration, many practical details will be described in the following description. However, it should be understood that the present disclosure should not be limited by these practical details. In other words, in some embodiments of the present disclosure, these practical details are unnecessary. In addition, in order to simplify the drawings, some conventionally used structures and components will be shown in simple schematic ways in the drawings.

Due to different vital signs of different populations, evaluation standards for normal values are slightly different. For example, the myocardial microcirculation function of the elderly is relatively poor, and the blood flow velocity is generally lower than that of the young. If the industry general evaluation standard is used, the blood flow velocity used will be higher than the actual value. The higher blood flow velocity will further affect the coronary artery evaluation parameters, such as: fractional flow reserve FFR, fractional flow reserve during a diastolic phase iFR, and index for microcirculatory resistance iFMR during the diastolic phase.

Therefore, at this stage, how to obtain an adjustment parameter according to individual differences, for improving the accuracy of a blood vessel calculation parameter, such as a blood flow velocity, has become an urgent problem in the field of coronary artery technology.

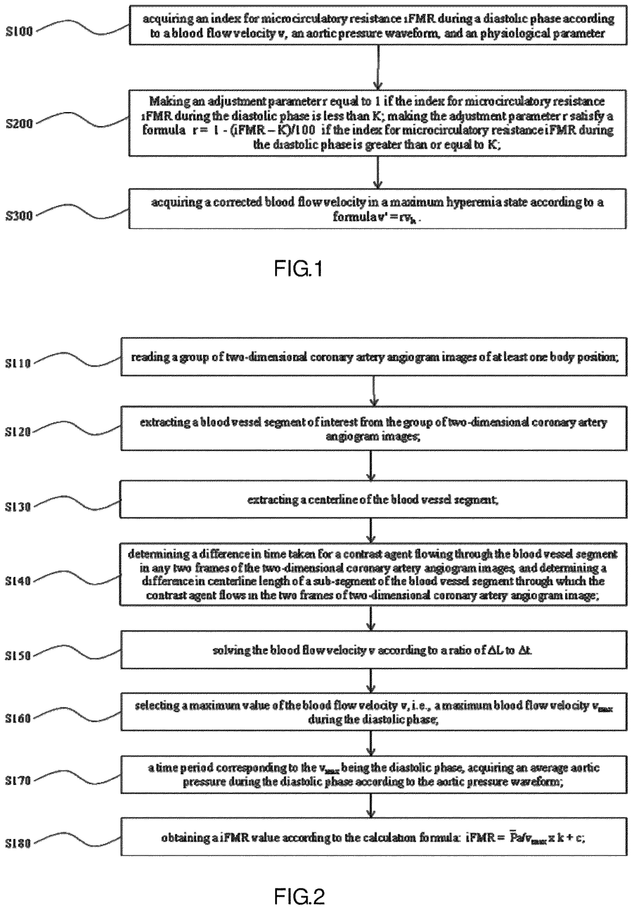

Embodiment 1: In order to solve the above problem, as shown in FIG. 1, the present disclosure provides a method for calculating an adjusted blood flow velocity in maximum hyperemia state based on index for microcirculatory resistance, comprising:

> S100, acquiring an index for microcirculatory resistance iFMR during a diastolic phase according to a blood flow velocity v, an aortic pressure waveform, and an physiological parameter;
> S200, making an adjustment parameter r equal to 1 if the index for microcirculatory resistance iFMR during the diastolic phase is less than K; making the adjustment parameter r satisfy a formula
> $$r = 1 - \frac{\text{iFMR} - K}{100}$$
> if the index for microcirculatory resistance iFMR during the diastolic phase is greater than or equal to K, wherein K is a positive number less than 100;
> S300, acquiring a corrected blood flow velocity in a maximum hyperemia state according to a formula $v'$

$= rv_h$;

wherein $v'$ represents the corrected blood flow velocity in the maximum hyperemia state, and $v_h$ represents a blood flow velocity in the maximum hyperemia state.

In an embodiment of the present disclosure, $v_h = z\bar{v} + x$;

wherein $v_h$ represents the blood flow velocity in the maximum hyperemia state, $\bar{v}$ represents an average blood flow velocity in a heartbeat cycle area, z is a constant in the range of 1 to 3, and x is a constant in the range of 50 to 300; K=50.

According to the present disclosure, an index for microcirculatory resistance iFMR during a diastolic phase is acquired according to a blood flow velocity v, an aortic pressure waveform, and an physiological parameter; then an adjustment parameter is obtained by comparing iFMR with K, where the adjustment parameter varies for different values of iFMR, further, a differentiated parameter is obtained according to individualized differences, placing a solid foundation for the accuracy of a blood vessel calculation parameter, then, a corrected blood flow velocity in the maximum hyperemia state is obtained by a product of the adjustment parameter and the blood flow velocity in the maximum hyperemia state, allowing for more targeted, individualized and more accurate measurement results.

Embodiment 2:

The present disclosure provides a method for calculating an adjusted blood flow velocity in maximum hyperemia state based on index for microcirculatory resistance, comprising:

S100, acquiring an index for microcirculatory resistance iFMR during a diastolic phase according to a blood flow velocity v, an aortic pressure waveform, and an physiological parameter; wherein

> 1) as shown in FIG. 2, when acquiring the blood flow velocity through a two-dimensional angiogram image, a manner for acquiring the blood flow velocity v comprises:

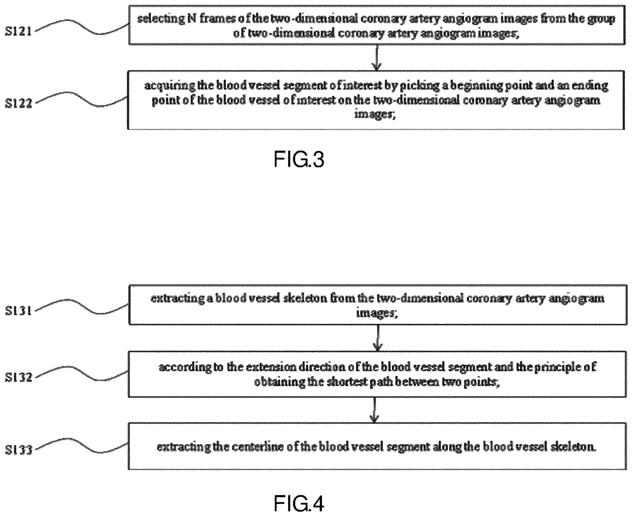

> > S110, reading a group of two-dimensional coronary artery angiogram images of at least one body position;
> > S120, extracting a blood vessel segment of interest from the group of two-dimensional coronary artery angiogram images, for which a specific manner is shown in FIG. 3, comprising:
> > S121, selecting N frames of the two-dimensional coronary artery angiogram images from the group of two-dimensional coronary artery angiogram images;
> > S122, acquiring the blood vessel segment of interest by picking a beginning point and an ending point of the blood vessel of interest on the two-dimensional coronary artery angiogram images;

S130, extracting a centerline of the blood vessel segment, for which a specific manner is shown in FIG. 4, comprising:

S131, extracting a blood vessel skeleton from the two-dimensional coronary artery angiogram images;

S132, according to the extension direction of the blood vessel segment and the principle of obtaining the shortest path between two points;

S133, extracting the centerline of the blood vessel segment along the blood vessel skeleton.

S140, determining a difference in time taken for a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$;

S150, solving the blood flow velocity according to a ratio of $\Delta L$ to $\Delta t$.

2) A manner for acquiring an aortic pressure waveform comprises:

acquiring, in real time, an aortic pressure $P_{aj}$ with an invasive blood pressure sensor or non-invasive blood pressure instrument, where j is a positive integer greater than or equal to 1, and then generating an aortic pressure waveform according to time;

3) A manner for acquiring the index for microcirculatory resistance iFMR during the diastolic phase comprises:

S160, selecting a maximum value of the blood flow velocity v obtained by solving in S150, i.e., a maximum blood flow velocity $v_{max}$ during the diastolic phase;

S170, a time period corresponding to the $v_{max}$ being the diastolic phase, acquiring an average aortic pressure during the diastolic phase according to the aortic pressure waveform, namely:

$$\overline{P}_a = \frac{\sum_1^j\left(P_{a1} + P_{a2} \dots P_{aj}\right)}{j} \; ;$$

S180, obtaining a iFMR value according to the calculation formula:

$$\mathrm{iFMR} = \overline{P}_a \big/ v_{\max} \times k + c \; ;$$

wherein, $\overline{P}_a$ represents the average aortic pressure during the diastolic phase; $P_{a1}$, $P_{a2}$, and $P_{aj}$ represent aortic pressures corresponding to a first point, a second point, and a j-th point within the diastolic phase on the aortic pressure waveform, respectively, and j represents the number of pressure points contained in the aortic pressure waveform during the diastolic phase, $v_h$ represents the blood flow velocity in the maximum hyperemia state obtained by selecting a maximum value from all blood flow velocities v, preferably by selecting a maximum value of blood flow velocity through a recursive algorithm or a bubbling algorithm; k and c represent the influence parameters k=1~3, c=0~10;

S200, making an adjustment parameter r equal to 1 if the index for microcirculatory resistance iFMR during the diastolic phase is less than K; making the adjustment parameter r satisfy a formula

$$r = 1 - \frac{\mathrm{iFMR} - K}{100}$$

if the index for microcirculatory resistance iFMR during the diastolic phase is greater than or equal to K, wherein K is a positive number less than 100; preferably, K=50.

All the contents of acquiring coronary artery blood vessel evaluation parameter based on the physiological parameter are within the scope of protection of the present disclosure. After a large number of experimental verifications, histories of having hypertension and diabetes and gender all have impacts on the accuracy of calculating a coronary artery blood vessel evaluation parameter. Therefore, in an embodiment of the present disclosure, an influence parameter k in S180 is calculated by the formula: k=a×b, where a represents a characteristic value of diabetes, b represents a characteristic value of hypertension, and c represents the gender. If a patient does not suffer from diabetes, then $0.5 \leq a \leq 1$, preferably, a=1; if the patient suffers from diabetes, then $1 < a \leq 2$, preferably, a=2; if the patient's blood pressure value is greater than or equal to 90 mmHg, then $1 < b \leq 1.5$, preferably, b=1.5; if the patient's blood pressure value is less than 90 mmHg, then $0.5 \leq b \leq 1$, preferably, b=1; if the patient is male, then c=0; if the patient is female, then c =3~10, preferably, c=5.

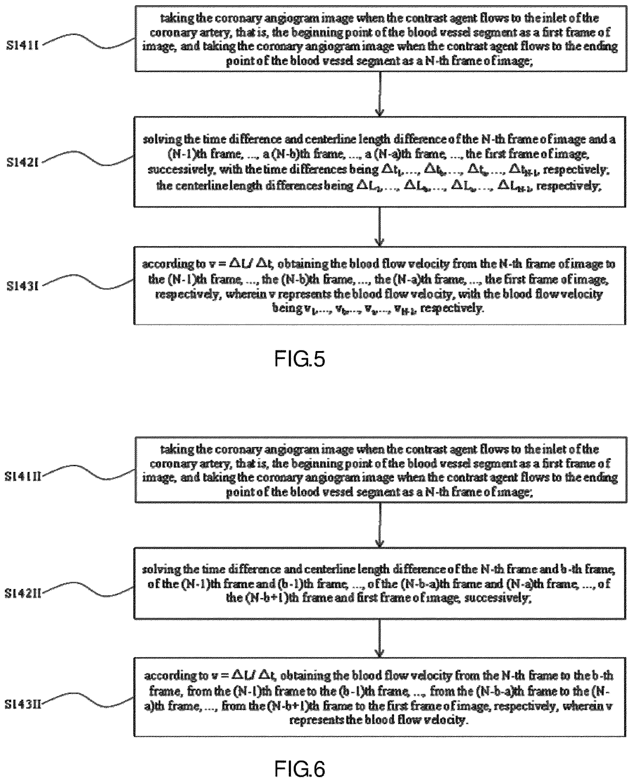

In an embodiment of the present disclosure, S140 comprises two acquisition methods. As shown in FIG. 5, method(1) comprises:

S141l, taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;

S142I, solving the time difference and centerline length difference of the N-th frame of image and a (N-1)th frame, ..., a (N-b)th frame, ..., a (N-a)th frame, ..., the first frame of image, successively, with the time differences being $\Delta t_1,..., \Delta t_b,..., \Delta t_a,..., \Delta t_{N-1}$, respectively; the centerline length differences being $\Delta L_1,..., \Delta L_b,..., \Delta L_a,..., \Delta L_{N-1}$, respectively;

S143I, according to $v = \Delta L / \Delta t$ , obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1,..., v_b,..., v_a,..., v_{N-1}$, respectively.

In an embodiment of the present disclosure, S140 comprises two acquisition methods. As shown in FIG. 6, method(2) comprises:

S141II, taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;

S142II, solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image, successively;

S14311, according to $v = \Delta L / \Delta t$ , obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

Embodiment 3:

In an embodiment of the present disclosure, a manner for acquiring a blood flow velocity by three-dimensional modeling in S100 comprises:

Step A, reading a group of two-dimensional coronary artery angiogram images of at least two body positions;

Step B, extracting a blood vessel segment of interest from the groups of two-dimensional coronary artery angiogram images;

Step C, acquiring geometric structure information of the blood vessel segment and extracting a centerline of the blood vessel segment;

Step D, performing graphics processing on the blood vessel segment of interest;

Step E, extracting a blood vessel contour line of the blood vessel segment;

Step F, according to the geometric structure information of the blood vessel segment, synthesizing a three-dimensional blood vessel model by projecting the at least two body positions' two-dimensional coronary angiogram images which have been extracted centerline and contour line of the blood vessel onto a three-dimensional plane;

Step G, determining a difference in time taken for a contrast agent flowing through the blood vessel segment in any two frames of the two-dimensional coronary artery angiogram images with the difference being $\Delta t$; according to the three-dimensional blood vessel model, acquiring a centerline of the three-dimensional blood vessel model, correcting the centerline extracted from the two-dimensional coronary angiogram images, and determining a difference in the corrected centerline length of a sub-segment of the blood vessel segment through which the contrast agent flows in the two frames of two-dimensional coronary artery angiogram image with the difference being $\Delta L'$; solving the blood flow velocity v according to the ratio of the $\Delta L'$ to the $\Delta t$.

$\Delta t = m \times fps$, since each group of two-dimensional coronary artery angiogram images contains multiple frames of two-dimensional coronary artery angiogram images played consecutively, m represents a difference in frame number between two frames of two-dimensional angiogram image selected from each group of two-dimensional coronary artery angiogram images, and fps represents an interval time for switching between two adjacent frames of the image, preferably, fps=1/15 second.

Embodiment 4:

The present disclosure provides a method for calculating an adjusted blood flow velocity in maximal hyperemia state based on physiological parameter, comprising:

the above method for calculating an adjusted blood flow velocity in maximum hyperemia state based on index for microciruulatory resistance;
according to the formula $v' = rv_h$;

$$v_h = z\overline{v} + x;$$

$$r = 1 - \frac{\text{iFMR} - K}{100}; \quad \text{iFMR} = \overline{P_a}/v_{\max} \times k + c.$$

wherein v' represents a blood flow velocity in the maximum hyperemia state which has been adjusted by a physiological parameter, $v_h$ represents a blood flow velocity in the maximum hyperemia state, v represents an average blood flow velocity in a heartbeat cycle area, z is a constant in the range of 1 to 3, and x is a constant in the range of 50 to 300.

An embodiment of the present disclosure provides a method for acquiring coronary artery blood vessel evaluation parameter based on physiological parameter, comprising the above method for acquiring blood flow velocity in maximal hyperemia state based on physiological parameter. A coronary artery blood vessel evaluation parameter comprises: fractional flow reserve FFR, index for microcirculatory resistance IMR, index for microcirculatory resistance iFMR during a diastolic phase, fractional flow reserve iFR during the diastolic phase and the like.

Embodiment 5:

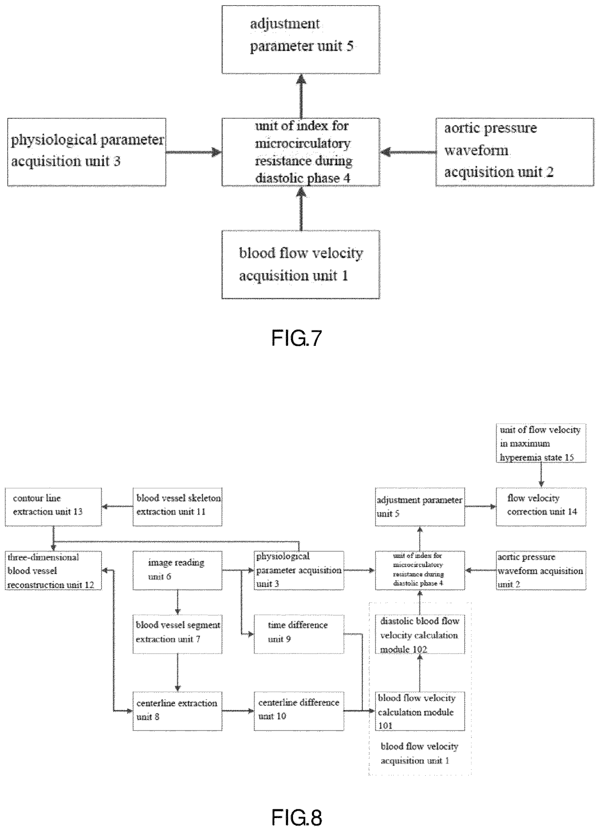

As shown in FIG. 7 , the present disclosure provides an apparatus for calculating an adjusted blood flow velocity in maximum hyperemia state based on index for microcirculatory resistance, which is used for the above method for calculating an adjusted blood flow velocity in maximum hyperemia state based on index for microcirculatory resistance, comprising: a blood flow velocity acquisition unit 1, an aortic pressure waveform acquisition unit 2, a physiological parameter acquisition unit 3, a unit of index for microcirculatory resistance during diastolic phase 4 and an adjustment parameter unit 5. The unit of index for microcirculatory resistance during diastolic phase 4 is connected with the blood flow velocity acquisition unit 1, the aortic pressure waveform acquisition unit 2 and the physiological parameter acquisition unit 3. The blood flow velocity acquisition unit 1 is configured to acquire a blood flow velocity v. The aortic pressure waveform acquisition unit 2 is configured to acquire, in real time, an aortic pressure waveform changing over time. The physiological parameter acquisition unit 3 is configured to acquire physiological parameters of a patient, comprising gender and disease history. The unit of index for microcirculatory resistance during diastolic phase 4 is configured to receive the blood flow velocity v, the aortic pressure waveform, and the physiological parameters sent by the blood flow velocity acquisition unit 1, the aortic pressure waveform acquisition unit 2, and the physiological parameter acquisition unit 3, and then to obtain an index for microcirculatory resistance iFMR during a diastolic phase according to the blood flow velocity v, the aortic pressure waveform, and the physiological parameters. The adjustment parameter unit 5 is configured to receive iFMR value of the unit of index for microcirculatory resistance during diastolic phase 4. An adjustment parameter r is equal to 1 if the index for microcirculatory resistance during the diastolic phase iFMR<K; the adjustment parameter r satisfies a formula

$$r = 1 - \frac{\text{iFMR} - K}{100}$$

if the index for microcirculatory resistance during the diastolic phase iFMR≥K; where K is a positive number less than 100.

As shown in FIG. 8, an embodiment of the present disclosure further comprises: a flow velocity correction unit 14 connected to the adjustment parameter unit 5, a unit of flow velocity in maximum hyperemia state 15 connected to the flow velocity correction unit 14. The unit of flow velocity in maximum hyperemia state 15 is configured to calculate a blood flow velocity in a maximum hyperemia state according to $v_h = z\overline{v}+x$; $v_h$ represents the blood flow velocity in the maximum hyperemia state, v represents an average blood flow velocity in a heartbeat cycle area, z is a constant in the range of 1 to 3, and x is a constant in the range of 50 to 300.

As shown in FIG. 8, an embodiment of the present application further comprises: an image reading unit 6, a blood vessel segment extraction unit 7, and a centerline extraction unit 8 connected in sequence, a time difference unit 9 and the physiological parameter acquisition unit 3 connected to the image reading unit 6, and the blood flow velocity acquisition unit 1 respectively connected with the time difference unit 9 and a centerline difference unit 10. The centerline difference unit 10 is connected with the centerline extraction unit 8. The image reading unit 6 is configured to read a group of two-dimensional coronary artery angiogram image of at least one body position. The blood vessel segment extraction unit 7 is configured to receive two-dimensional coronary artery angiogram images sent by the image reading unit 6, and to extract a blood vessel segment of interest in the images. The centerline extraction unit 8 is configured to receive the blood vessel segment sent by the blood vessel segment extraction unit 7, and to extract the centerline of the blood vessel segment. The time difference unit 9 is configured to receive any two frames of the two-dimensional coronary artery angiogram images sent by the image reading unit 6, and to determine a difference in time taken for a contrast agent flowing through the blood vessel segment in the two frames of two-dimensional coronary artery angiogram image with the difference being $\Delta t$. The centerline difference unit 10 is configured to receive the centerline of a sub-segment of the blood vessel segment flowed through by the contrast agent in the two frames of two-dimensional coronary artery angiogram image sent by the centerline extraction unit, and to determine a difference in centerline length of the sub-segment of the blood vessel segment through which the contrast agent flows in the two frames of two-dimensional coronary artery angiogram image with the difference being $\Delta L$. The blood flow velocity acquisition unit 1 comprises a blood flow velocity calculation module 101 and a diastolic blood flow velocity calculation module 102. The blood flow velocity calculation module 101 is respectively connected to the time difference unit 9 and the centerline difference unit 10. The diastolic blood flow velocity calculation module 102 is connected with the blood flow velocity calculation module 101. The blood flow velocity calculation module 101 is configured to receive the $\Delta L$ and the $\Delta t$ sent by the time difference unit 9 and the centerline difference unit 10, and to solve the blood flow velocity according to the ratio of $\Delta L$ to $\Delta t$. The diastolic blood flow velocity calculation module 102 is configured to receive the blood flow velocity sent by the blood flow velocity calculation module 101, and to select a maximum value of the blood flow velocity as a blood flow velocity during a diastolic phase. The physiological parameter acquisition unit 3 is configured to receive the two-dimensional coronary artery angiogram images of the image reading unit 6, to acquire a physiological parameter of a patient, image shooting angles and imaging distance, and to transmit the physiological parameter, image shooting angles and imaging distance to the unit of index for microcirculatory resistance during diastolic phase 4.

The above imaging distance may be understood as: when synthesizing a three-dimensional model by two plane images, as long as the distance between the object and the imaging plane, the image shooting angle, and the two two-dimensional plane images are known, the three-dimensional model can be generated through the principle of three-dimensional imaging.

In an embodiment of the present application, the apparatus further comprises: a blood vessel skeleton extraction unit 11 and a three-dimensional blood vessel reconstruction unit 12, both connected to the image reading unit 6, a contour line extraction unit 13 connected to the blood vessel skeleton extraction unit 11. The three-dimensional blood vessel reconstruction unit 12 is connected with the physiological parameter acquisition unit 3, the centerline extraction unit 8 and the contour line extraction unit 13. The blood vessel skeleton extraction unit 11 is configured to receive the two-dimensional coronary artery angiogram images sent by the image reading unit 6, and to extract a blood vessel skeleton in the images. The contour line extraction unit 13 is configured to receive the blood vessel skeleton of the blood vessel skeleton extraction unit 11, and to extract a contour line of the blood vessel segment of interest according to the blood vessel skeleton. The three-dimensional blood vessel reconstruction unit 12 is configured to receive the contour line, the image shooting angles and the centerline sent by the contour line extraction unit 13, the physiological parameter acquisition unit 3 and the centerline extraction unit 8, and to receive the two-dimensional coronary artery angiogram images sent by the image reading unit 6 in order to synthesize a three-dimensional blood vessel model by projecting at least two body positions' two-dimensional coronary angiogram images which have been extracted centerline and contour line of the blood vessel onto a three-dimensional plane according to the geometric structure information of the blood vessel segment. The centerline extraction unit 8 is configured to re-extract the centerline of the blood vessel segment from the three-dimensional blood vessel model of the three-dimensional blood vessel reconstruction unit 12, and to re-acquire the length of the centerline.

The present disclosure provides a coronary artery analysis system, which comprises the apparatus for calculating an adjusted blood flow velocity in maximum hyperemia state based on index for microcirculatory resistance according to any one of the above.

The present disclosure provides a computer storage medium having stored thereon a computer program to be executed by a processor, wherein the above method for calculating an adjusted blood flow velocity in maximum hyperemia state based on index for microcirculatory resistance is implemented when the computer program is executed by the processor.

A person skilled in the art knows that various aspects of the present disclosure can be implemented as a system, a method, or a computer program product. Therefore, each aspect of the present disclosure can be specifically implemented in the following forms, namely: complete hardware implementation, complete software implementation (including firmware, resident software, microcode, etc.), or a combination of hardware and software implementations, which here can be collectively referred to as "circuit", "module" or "system". In addition, in some embodiments, various aspects of the present disclosure may also be implemented in the form of a computer program product in one or more computer-readable media, and the computer-readable medium contains computer-readable program code. Implementation of a method and/or a system of embodiments of the present disclosure may involve performing or completing selected tasks manually, automatically, or a combination thereof.

For example, hardware for performing selected tasks according to the embodiment(s) of the present disclosure may be implemented as a chip or a circuit. As software, selected tasks according to the embodiment(s) of the present disclosure can be implemented as a plurality of software instructions executed by a computer using any suitable operating system. In the exemplary embodiment(s) of the present disclosure, a data processor performs one or more tasks according to the exemplary embodiment(s) of a method and/or system as described herein, such as a computing platform for executing multiple instructions. Optionally, the data processor comprises a volatile memory for storing instructions and/or data, and/or a non-volatile memory for storing instructions and/or data, for example, a magnetic hard disk and/or movable medium. Optionally, a network connection is also provided. Optionally, a display and/or user input device, such as a keyboard or mouse, are/is also provided.

Any combination of one or more computer readable media can be utilized. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination of the above. More specific examples (non-exhaustive list) of computer-readable storage media would include the following:

Electrical connection with one or more wires, portable computer disk, hard disk, random access memory (RAM), read only memory (ROM), erasable programmable read only memory (EPROM or flash memory), optical fiber, portable compact disk read only memory (CD-ROM), optical storage device, magnetic storage device, or any suitable combination of the above. In this document, the computer-readable storage medium can be any tangible medium that contains or stores a program, and the program can be used by or in combination with an instruction execution system, apparatus, or device.

The computer-readable signal medium may include a data signal propagated in baseband or as a part of a carrier wave, which carries computer-readable program code. This data signal for propagation can take many forms, including but not limited to electromagnetic signals, optical signals, or any suitable combination of the above. The computer-readable signal medium may also be any computer-readable medium other than the computer-readable storage medium. The computer-readable medium can send, propagate, or transmit a program for use by or in combination with the instruction execution system, apparatus, or device.

The program code contained in the computer-readable medium can be transmitted by any suitable medium, including, but not limited to, wireless, wired, optical cable, RF, etc., or any suitable combination of the above.

For example, any combination of one or more programming languages can be used to write computer program codes for performing operations for various aspects of the present disclosure, including object-oriented programming languages such as Java, Smalltalk, C++, and conventional process programming languages, such as "C" programming language or similar programming language. The program code can be executed entirely on a user's computer, partly on a user's computer, executed as an independent software package, partly on a user's computer and partly on a remote computer, or entirely on a remote computer or server. In the case of a remote computer, the remote computer can be connected to a user's computer through any kind of network including a local area network (LAN) or a wide area network (WAN), or it can be connected to an external computer (for example, connected through Internet provided by an Internet service provider).

It should be understood that each block of the flowcharts and/or block diagrams and combinations of blocks in the flowcharts and/or block diagrams can be implemented by computer program instructions. These computer program instructions can be provided to the processor of general-purpose computers, special-purpose computers, or other programmable data processing devices to produce a machine, which produces a device that implements the functions/actions specified in one or more blocks in the flowcharts and/or block diagrams when these computer program instructions are executed by the processor of the computer or other programmable data processing devices.

It is also possible to store these computer program instructions in a computer-readable medium. These instructions make computers, other programmable data processing devices, or other devices work in a specific manner, so that the instructions stored in the computer-readable medium generate an article of manufacture comprising instructions for implementation of the functions/actions specified in one or more blocks in the flowcharts and/or block diagrams.

Computer program instructions can also be loaded onto a computer (for example, a coronary artery analysis system) or other programmable data processing equipment to facilitate a series of operation steps to be performed on the computer, other programmable data processing apparatus or other apparatus to produce a computer-implemented process, which enable instructions executed on a computer, other programmable device, or other apparatus to provide a process for implementing the functions/actions specified in the flowcharts and/or one or more block diagrams.

The above specific examples of the present disclosure further describe the purpose, technical solutions and beneficial effects of the present disclosure in detail. It should be understood that the above are only specific embodiments of the present disclosure and are not intended to limit the present disclosure. The invention is defined by the appended claims.

**Claims**

1. A computer-implemented method for calculating an adjusted blood flow velocity in maximum hyperemia state based on index for microcirculatory resistance, **characterized by** comprising:

    acquiring an index for microcirculatory resistance iFMR during a diastolic phase according to a blood flow velocity v, an aortic pressure waveform, and an physiological parameter; making an adjustment parameter r equal to 1 if the index for microcirculatory resistance iFMR during the diastolic phase is less than K; making the adjustment parameter r satisfy a formula

$$r = 1 - \dfrac{\text{iFMR} - K}{100}$$

if the index for microcirculatory resistance iFMR during the diastolic phase is greater than or equal to K, wherein K is a positive number less than 100;

acquiring a corrected blood flow velocity in a maximum hyperemia state according to a formula $v' = rv_h$;

wherein $v'$ represents the corrected blood flow velocity in the maximum hyperemia state, and $v_h$ represents a blood flow velocity in the maximum hyperemia state.

2. The method for calculating an adjusted blood flow velocity in maximum hyperemia state based on index for microcirculatory resistance according to claim 1, **characterized in that**,

$$v_h = z\overline{v} + x$$

wherein $v_h$ represents the blood flow velocity in the maximum hyperemia state, $\overline{v}$ represents an average blood flow velocity in a heartbeat cycle area, z is a constant in the range of 1 to 3, and x is a constant in the range of 50 to 300; K=50.

3. The method for calculating an adjusted blood flow velocity in maximum hyperemia state based on index for microcirculatory resistance according to claim 1, **characterized in that**, a manner for acquiring an index for microcirculatory resistance iFMR during a diastolic phase according to a blood flow velocity v, an aortic pressure waveform, and an physiological parameter comprises:

selecting a maximum value of the blood flow velocity v, i.e., a maximum blood flow velocity $v_{max}$ during the diastolic phase;
a time period corresponding to the $v_{max}$ being the diastolic phase, acquiring an average aortic pressure during the diastolic phase according to the aortic pressure waveform;

$$\text{iFMR} = \overline{P_a} / v_{max} \times k + c\,;$$

$$\overline{P_a} = \dfrac{\sum_1^j \left( P_{a1} + P_{a2} \ldots P_{aj} \right)}{j}\,;$$

wherein, $\overline{P_a}$ represents the average aortic pressure during the diastolic phase; $P_{a1}$, $P_{a2}$, and $P_{aj}$ represent aortic pressures corresponding to a first point, a second point, and a j-th point within the diastolic phase on the aortic pressure waveform, respectively, and j represents the number of pressure points contained in the aortic pressure waveform during the diastolic phase, $v_h$ represents the blood flow velocity in the maximum hyperemia state obtained by selecting a maximum value from all blood flow velocities v; k and c represent the influence parameters k=1~3, c=0~10,

4. The method for calculating an adjusted blood flow velocity in maximum hyperemia state based on index for microcirculatory resistance according to claim 3, **characterized in that**, the influence parameter k=a×b, wherein a represents a characteristic value of diabetes, b represents a characteristic value of hypertension, and c represents gender.

5. The method for calculating an adjusted blood flow velocity in maximum hyperemia state based on index for microcirculatory resistance according to claim 4, **characterized in that**, if a patient does not suffer from diabetes, then $0.5 \leq a \leq 1$;

if the patient suffers from diabetes, then $1 < a \leq 2$;
if the patient's blood pressure is greater than or equal to 90 mmHg, then $1 < b \leq 1.5$; if the patient's blood pressure is less than 90 mmHg, then $0.5 \leq b \leq 1$;
if the patient is male, then c=0; if the patient is female, then c=3~10.

6. The method for calculating an adjusted blood flow velocity in maximum hyperemia state based on index for microcirculatory resistance according to claim 5, **characterized in that**, if the patient does not suffer from diabetes, then a=1; If the patient suffers from diabetes, then a=2;

if the patient's blood pressure is greater than or equal to 90 mmHg, then b=1.5; if the patient's blood pressure is less than 90 mmHg, then b=1;
if the patient is male, c=0; if the patient is female, c=5.

7. The method for calculating an adjusted blood flow velocity in maximum hyperemia state based on index for microcirculatory resistance according to claim 1, **characterized in that**, a manner for acquiring a blood flow velocity comprises:

reading a group of two-dimensional coronary artery angiogram images of at least one body position;
extracting a blood vessel segment of interest from the group of two-dimensional coronary artery angiogram images;

extracting a centerline of the blood vessel segment;

determining a difference in time taken for a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$ ;

solving the blood flow velocity according to a ratio of $\Delta L$ to $\Delta t$.

8. The method for calculating an adjusted blood flow velocity in maximum hyperemia state based on index for microcirculatory resistance according to claim 7, **characterized in that**, a manner for extracting a blood vessel segment of interest from the group of two-dimensional coronary artery angiogram images comprises:

selecting N frames of the two-dimensional coronary artery angiogram images from the group of two-dimensional coronary artery angiogram images;

acquiring the blood vessel segment of interest by picking a beginning point and an ending point of the blood vessel of interest on the two-dimensional coronary artery angiogram images.

9. The method for calculating an adjusted blood flow velocity in maximum hyperemia state based on index for microcirculatory resistance according to claim 7, **characterized in that**, a manner for extracting the centerline of the blood vessel segment comprises:

extracting a blood vessel skeleton from the two-dimensional coronary artery angiogram images;

according to an extension direction of the blood vessel segment and a principle of obtaining the shortest path between two points;

extracting the centerline of the blood vessel segment along the blood vessel skeleton.

10. The method for calculating an adjusted blood flow velocity in maximum hyperemia state based on index for microcirculatory resistance according to claim 7, **characterized in that**, a manner for determining a difference in time taken for a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference

being $\Delta L$ , and solving the blood flow velocity according to the ratio of $\Delta L$ to $\Delta t$ comprises:

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image; solving the time difference and centerline length difference of the N-th frame of image and a (N-1)th frame, ..., a (N-b)th frame, ..., a (N-a)th frame, ..., the first frame of image, successively, with the time differences being $\Delta t_1$,..., $\Delta t_b$,..., $\Delta t_a$,..., $\Delta t_{N-1}$, respectively; the centerline length differences being $\Delta L_1$,..., $\Delta L_b$,..., $\Delta L_a$,..., $\Delta L_{N-1}$, respectively;

according to $$v = \frac{\Delta L}{\Delta t}$$ , obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1$,..., $v_b$,..., $v_a$,..., $v_{N-1}$, respectively.

11. The method for calculating an adjusted blood flow velocity in maximum hyperemia state based on index for microcirculatory resistance according to claim 7, **characterized in that**, a manner for determining a difference in time taken a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$ , and solving the blood flow velocity according to the ratio of $\Delta L$ to $\Delta t$ comprises:

solving the time difference and centerline length difference of the N-th frame and b-th frame, of the (N-1)th frame and (b-1)th frame, ..., of the (N-b-a)th frame and (N-a)th frame, ..., of the (N-b+1)th frame and first frame of image, successively;

according to $$v = \frac{\Delta L}{\Delta t}$$ , obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

**12.** The method for calculating an adjusted blood flow velocity in maximum hyperemia state based on index for microcirculatory resistance according to claim 7, **characterized in that**, after the manner for extracting a centerline of the blood vessel segment, and before the manner for determining a difference in time taken for a contrast agent flowing through the blood vessel segment in any two frames of the two-dimensional coronary artery angiogram images with the difference being $\Delta t$, and determining a difference in centerline length of a sub-segment of the blood vessel segment through which the contrast agent flows in the two frames of two-dimensional coronary artery angiogram image with the difference being $\Delta L$, further comprises:

> reading a group of two-dimensional coronary artery angiogram images of at least two body positions;
> acquiring geometric structure information of the blood vessel segment;
> performing graphics processing on the blood vessel segment of interest;
> extracting a blood vessel contour line of the blood vessel segment;
> according to the geometric structure information of the blood vessel segment, synthesizing a three-dimensional blood vessel model by projecting the at least two body positions' two-dimensional coronary angiogram images which have been extracted centerline and contour line of the blood vessel onto a three-dimensional plane.

**13.** The method for calculating an adjusted blood flow velocity in maximum hyperemia state based on index for microcirculatory resistance according to claim 12, **characterized in that**, a manner for solving the blood flow velocity according to the ratio of $\Delta L$ to $\Delta t$ comprises:

> according to the three-dimensional blood vessel model, acquiring a centerline of the three-dimensional blood vessel model, correcting the centerline extracted from the two-dimensional coronary angiogram images, and correcting the centerline difference $\Delta L$ to obtain $\Delta L$';
> solving the blood flow velocity v according to the ratio of the $\Delta L$' to the $\Delta t$.

**14.** A method for acquiring coronary artery blood vessel evaluation parameter based on physiological parameter, **characterized by** comprising: the method for calculating an adjusted blood flow velocity in maximum hyperemia state based on index for microcirculatory resistance according to claim 13.

**15.** An apparatus for calculating an adjusted blood flow velocity in maximum hyperemia state based on index for microcirculatory resistance, configured to carry out the method for calculating an adjusted blood flow velocity in maximum hyperemia state based on index for microcirculatory resistance according to any one of the claims 1 to 13, **characterized by** comprising: a blood flow velocity acquisition unit (1), an aortic pressure waveform acquisition unit (2), a physiological parameter acquisition unit (3), a unit of index for microcirculatory resistance during diastolic phase (4) and an adjustment parameter unit (5); the unit of index for microcirculatory resistance during diastolic phase (4) being connected with the blood flow velocity acquisition unit (1), the aortic pressure waveform acquisition unit (2) and the physiological parameter acquisition unit (3);

> the blood flow velocity acquisition unit (1) being configured to acquire a blood flow velocity v;
> the aortic pressure waveform acquisition unit (2) being configured to acquire, in real time, an aortic pressure waveform changing over time;
> the physiological parameter acquisition unit (3) being configured to acquire physiological parameters of a patient, comprising gender and disease history;
> the unit of index for microcirculatory resistance during diastolic phase (4) being configured to receive the blood flow velocity v, the aortic pressure waveform, and the physiological parameters sent by the blood flow velocity acquisition unit (1), the aortic pressure waveform acquisition unit (2), and the physiological parameter acquisition unit (3), and then to obtain an index for microcirculatory resistance iFMR during a diastolic phase according to the blood flow velocity v, the aortic pressure waveform, and the physiological parameters;
> the adjustment parameter unit (5) being configured to receive iFMR value of the unit of index for microcirculatory resistance during diastolic phase (4); make an adjustment parameter r equal to 1 if the index for microcirculatory resistance during the diastolic phase iFMR<K; make the adjustment parameter r satisfy a formula

$$r = 1 - \frac{\text{iFMR} - K}{100}$$

> if the index for microcirculatory resistance during the diastolic phase iFMR≥K; where K is a positive number less than 100.

**16.** The apparatus for calculating an adjusted blood flow velocity in maximum hyperemia state based on index for microcirculatory resistance according to claim 15, **characterized by** further comprising: an image reading unit (6), a blood vessel segment extraction unit (7), and a centerline extraction unit (8) connected in sequence, a time difference unit (9) and the physiological parameter acquisition unit (3) connected to the image reading unit (6), the blood flow velocity acquisition unit (1) being connected with the time difference unit (9) and a centerline difference unit (10), respectively; the centerline difference unit (10) being connected with the centerline extraction unit (8);

the image reading unit (6) being configured to read a group of two-dimensional coronary artery angiogram image of at least one body position; the blood vessel segment extraction unit (7) being configured to receive two-dimensional coronary artery angiogram images sent by the image reading unit (6), and to extract a blood vessel segment of interest in the images; the centerline extraction unit (8) being configured to receive the blood vessel segment sent by the blood vessel segment extraction unit (7), and to extract the centerline of the blood vessel segment; the time difference unit (9) being configured to receive any two frames of the two-dimensional coronary artery angiogram images sent by the image reading unit (6), and to determine a difference in time taken for a contrast agent flowing through the blood vessel segment in the two frames of two-dimensional coronary artery angiogram image with the difference being $\Delta t$; the centerline difference unit (10) being configured to receive the centerline of a sub-segment of the blood vessel segment flowed through by the contrast agent in the two frames of two-dimensional coronary artery angiogram image sent by the centerline extraction unit, and to determine a difference in centerline length of the sub-segment of the blood vessel segment through which the contrast agent flows in the two frames of two-dimensional coronary artery angiogram image with the difference being $\Delta L$; the blood flow velocity acquisition unit (1), comprising a blood flow velocity calculation module (101) and a diastolic blood flow velocity calculation module (102), the blood flow velocity calculation module (101) being respectively connected to the time difference unit (9) and the centerline difference unit (10), the diastolic blood flow velocity calculation module (102) being connected with the blood flow velocity calculation module (101); the blood flow velocity calculation module (101) being configured to receive the $\Delta L$ and the $\Delta t$ sent by the time difference unit (9) and the centerline difference unit (10), and to solve the blood flow velocity according to the ratio of $\Delta L$ to $\Delta t$; the diastolic blood flow velocity calculation module (102) being configured to receive the blood flow velocity sent by the blood flow velocity calculation module (101), and to select a maximum value of the blood flow velocity as a blood flow velocity during a diastolic phase; the physiological parameter acquisition unit (3) being configured to receive the two-dimensional coronary artery angiogram images of the image reading unit (6), to acquire a physiological parameter of a patient and image shooting angles, and to transmit the physiological parameter and image shooting angles to the unit of index for microcirculatory resistance during diastolic phase (4).

**17.** The apparatus for calculating an adjusted blood flow velocity in maximum hyperemia state based on index of microcirculatory resistance according to claim 16, further comprising: a blood vessel skeleton extraction unit (11) and a three-dimensional blood vessel reconstruction unit (12), both connected to the image reading unit (6), a contour line extraction unit (13) connected to the blood vessel skeleton extraction unit (11), the three-dimensional blood vessel reconstruction unit (12) being connected with the physiological parameter acquisition unit (3), the centerline extraction unit (8) and the contour line extraction unit (13);

the blood vessel skeleton extraction unit (11) being configured to receive the two-dimensional coronary artery angiogram images sent by the image reading unit (6), and to extract a blood vessel skeleton in the images; the contour line extraction unit (13) being configured to receive the blood vessel skeleton of the blood vessel skeleton extraction unit (11), and to extract a contour line of the blood vessel segment of interest according to the blood vessel skeleton; the three-dimensional blood vessel reconstruction unit (12) being configured to receive the contour line, the image shooting angles and the centerline sent by the contour line extraction unit (13), the physiological parameter acquisition unit (3) and the centerline extraction unit (8), and to receive the two-dimensional coronary artery angiogram images sent by the image reading unit (6) in order to synthesize a three-dimensional blood vessel model by projecting the two-dimensional coronary angiogram images of at least two body positions with extracted centerline and contour line of the blood vessel onto a three-dimensional plane and according to the geometric structure information of the blood vessel segment; the centerline extraction unit (8) being configured to re-extract the centerline of the blood vessel segment from the three-dimensional blood vessel model of the three-dimensional blood vessel reconstruction unit (12), and to re-acquire the length of the centerline.

18. A coronary artery analysis system, **characterized by** comprising: the apparatus for calculating an adjusted blood flow velocity in maximum hyperemia state based on index for microcirculatory resistance according to any one of claims 15 to 17.

19. A computer storage medium having stored thereon a computer program to be executed by a processor, **characterized in that**, the method for calculating an adjusted blood flow velocity in maximum hyperemia state based on index for microcirculatory resistance according to any one of claims 1 to 13 is implemented when the computer program is executed by the processor.

**Patentansprüche**

1. Computerimplementiertes Verfahren zum Berechnen einer angepassten Blutströmungsgeschwindigkeit im maximalen Hyperämiezustand basierend auf einem Mikrozirkulationswiderstandsindex, **dadurch gekennzeichnet, dass** es umfasst:

Erhalten eines Mikrozirkulationswiderstandsindexes iFMR während einer diastolischen Phase gemäß einer Blutströmungsgeschwindigkeit v, einer Aortendruckwellenform und einem physiologischen Parameter;
Machen, dass ein Anpassungsparameter r gleich 1 ist, wenn der Mikrozirkulationswiderstandsindex iFMR während der diastolischen Phase kleiner als K ist;
Machen, dass der Anpassungsparameter r eine Formel $r = 1 - \dfrac{\text{iFMR} - K}{100}$ erfüllt, wenn der Mikrozirkulationswiderstandsindex iFMR während der diastolischen Phase größer als oder gleich K ist, wobei K eine positive Zahl kleiner als 100 ist;
Erhalten einer korrigierten Blutströmungsgeschwindigkeit in einem maximalen Hyperämiezustand gemäß einer Formel $v' = rv_h$;
wobei $v'$ die korrigierte Blutströmungsgeschwindigkeit in dem maximalen Hyperämiezustand darstellt und $v_h$ eine Blutströmungsgeschwindigkeit in dem maximalen Hyperämiezustand darstellt.

2. Verfahren zum Berechnen einer angepassten Blutströmungsgeschwindigkeit im maximalen Hyperämiezustand basierend auf einem Mikrozirkulationswiderstandsindex nach Anspruch 1, **dadurch gekennzeichnet, dass**

$$v_h = zv + x$$

wobei $v_h$ die Blutströmungsgeschwindigkeit in dem maximalen Hyperämiezustand darstellt, $\bar{v}$ eine durchschnittliche Blutströmungsgeschwindigkeit in einem Herzschlagzyklusbereich darstellt, z eine Konstante im Bereich von 1 bis 3 ist und x eine Konstante im Bereich von 50 bis 300 ist; K = 50.

3. Verfahren zum Berechnen einer angepassten Blutströmungsgeschwindigkeit im maximalen Hyperämiezustand basierend auf einem Mikrozirkulationswiderstandsindex nach Anspruch 1, **dadurch gekennzeichnet, dass** eine Art des Erhaltens eines Mikrozirkulationswiderstandsindexes iFMR während einer diastolischen Phase gemäß einer Blutströmungsgeschwindigkeit v, einer Aortendruckwellenform und einem physiologischen Parameter umfasst:

Auswählen eines maximalen Werts der Blutströmungsgeschwindigkeit v, d.h. einer maximalen Blutströmungsgeschwindigkeit $v_{max}$ während der diastolischen Phase;
wenn eine Zeitspanne entsprechend $v_{max}$ die diastolische Phase ist, Erhalten eines durchschnittlichen Aortendrucks während der diastolischen Phase gemäß der Aortendruckwellenform;

$$\text{iFMR} = \overline{P_a}/v_{max} \times k + c\,;$$

$$\overline{P_a} = \frac{\sum_1^j \left(P_{a1} + P_{a2} \ldots P_{aj}\right)}{j}\,;$$

wobei $\overline{P_a}$ den durchschnittlichen Aortendruck während der diastolischen Phase darstellt; $P_{a1}$, $P_{a2}$, und $P_{aj}$ Aortendrucke darstellen, die jeweils einem ersten Punkt, einem zweiten Punkt und einem j. Punkt innerhalb der diastolischen Phase auf der Aortendruckwellenform entsprechen, und j die Anzahl von Druckpunkten darstellt, die in der Aortendruckwellenform während der diastolischen Phase enthalten sind, $v_h$ die Blutströmungsgeschwindigkeit im maximalen Hyperämiezustand darstellt, die durch Auswählen eines maximalen Werts aus allen Blutströmungsgeschwindigkeiten v erlangt wird; k und c die Einflussparameter k=1~3, c=0~10 darstellen.

4. Verfahren zum Berechnen einer angepassten Blutströmungsgeschwindigkeit im maximalen Hyperämiezustand basierend auf einem Mikrozirkulationswiderstandsindex nach Anspruch 3, **dadurch gekennzeichnet, dass** der Einflussparameter k=a×b ist, wobei a einen charakteristischen Wert von Diabetes darstellt, b einen charakteristischen Wert von Hypertonie darstellt und c ein Geschlecht darstellt.

5. Verfahren zum Berechnen einer angepassten Blutströmungsgeschwindigkeit im maximalen Hyperämiezustand basierend auf einem Mikrozirkulationswiderstandsindex nach Anspruch 4, **dadurch gekennzeichnet, dass**, wenn ein Patient nicht an Diabetes leidet, 0,5≤a≤1 ist; wenn der Patient an Diabetes leidet, dann 1<a≤2 ist;

> wenn der Blutdruck des Patienten größer als oder gleich 90 mmHg ist, dann 1<b≤1,5 ist; wenn der Blutdruck des Patienten kleiner als 90 mmHg ist, dann 0,5≤b≤1 ist; wenn der Patient männlich ist, dann c=0 ist; wenn der Patient weiblich ist, dann c=3~10 ist.

6. Verfahren zum Berechnen einer angepassten Blutströmungsgeschwindigkeit im maximalen Hyperämiezustand basierend auf einem Mikrozirkulationswiderstandsindex nach Anspruch 5, **dadurch gekennzeichnet, dass**, wenn der Patient nicht an Diabetes leidet, dann a=1 ist; wenn der Patient an Diabetes leidet, dann a=2 ist;

> wenn der Blutdruck des Patienten größer als oder gleich 90 mmHg ist, dann b=1,5 ist; wenn der Blutdruck des Patienten kleiner als 90 mmHg ist, dann b=1 ist; wenn der Patient männlich ist, c=0 ist; wenn der Patient weiblich ist, c=5 ist.

7. Verfahren zum Berechnen einer angepassten Blutströmungsgeschwindigkeit im maximalen Hyperämiezustand basierend auf einem Mikrozirkulationswiderstandsindex nach Anspruch 1, **dadurch gekennzeichnet, dass** eine Art zum Erhalten einer Blutströmungsgeschwindigkeit umfasst:

> Auslesen einer Gruppe von zweidimensionalen Koronararterien-Angiogrammbildern mindestens einer Körperposition; Extrahieren eines Blutgefäßsegments von Interesse aus der Gruppe von zweidimensionalen Koronararterien-Angiogrammbildern; Extrahieren einer Mittellinie des Blutgefäßsegments; Bestimmen einer Zeitdifferenz, die für ein Kontrastmittel genommen wird, das durch das Blutgefäßsegment fließt, in zwei beliebigen Rahmen der zweidimensionalen Koronararterien-Angiogrammbilder mit der Differenz von $\varDelta t$, und Bestimmen einer Differenz der Mittellinienlänge eines Untersegments des Blutgefäßsegments, durch das das Kontrastmittel fließt, in den beiden Rahmen des zweidimensionalen Koronararterien-Angiogrammbildes mit der Differenz von $\varDelta L$ ; Lösen der Blutströmungsgeschwindigkeit gemäß einem Verhältnis von $\varDelta L$. zu $\varDelta t$.

8. Verfahren zum Berechnen einer angepassten Blutströmungsgeschwindigkeit im maximalen Hyperämiezustand basierend auf einem Mikrozirkulationswiderstandsindex nach Anspruch 7, **dadurch gekennzeichnet, dass** eine Art des Extrahierens eines Blutgefäßsegments von Interesse aus der Gruppe von zweidimensionalen Koronararterien-Angiogrammbildern umfasst:

> Auswählen von N Rahmen der zweidimensionalen Koronararterien-Angiogrammbilder aus der Gruppe von zweidimensionalen Koronararterien-Angiogrammbildern; Erhalten des Blutgefäßsegments von Interesse durch Wählen eines Anfangspunkts und eines Endpunkts des Blutgefäßes von Interesse auf den zweidimensionalen Koronararterien-Angiogrammbildern.

9. Verfahren zum Berechnen einer angepassten Blutströmungsgeschwindigkeit im maximalen Hyperämiezustand basierend auf einem Mikrozirkulationswiderstandsindex nach Anspruch 7, **dadurch gekennzeichnet, dass** eine Art des Extrahierens der Mittellinie des Blutgefäßsegments umfasst:

> Extrahieren eines Blutgefäßskeletts aus den zweidimensionalen Koronararterien-Angiogrammbildern; gemäß einer Erstreckungsrichtung des Blutgefäßsegments und einem Prinzip des Erlangens des kürzesten Pfads zwischen zwei Punkten; Extrahieren der Mittellinie des Blutgefäßsegments entlang des Blutgefäßskeletts.

10. Verfahren zum Berechnen einer angepassten Blutströmungsgeschwindigkeit im maximalen Hyperämiezustand basierend auf einem Mikrozirkulationswiderstandsindex nach Anspruch 7, **dadurch gekennzeichnet, dass** eine Art des Bestimmens einer Zeitdifferenz, die für ein Kontrastmittel genommen wird, das durch das Blutgefäßsegment fließt, in zwei beliebigen Rahmen der zweidimensionalen Koronararterien-Angiogrammbilder mit der Differenz von $\varDelta t$, und des Bestimmens einer Differenz der Mittellinienlänge eines Untersegments des Blutgefäßsegments, durch das das Kontrastmittel fließt, in den beiden Rahmen des zweidimensionalen Koronararterien-Angiogrammbildes mit der Differenz von $\Delta L$ und das Lösen der Blutströmungsgeschwindigkeit gemäß dem Verhältnis von $\Delta L$ zu $\Delta t$ umfasst:

Aufnehmen des Koronar-Angiogrammbildes, wenn das Kontrastmittel zum Einlass der Koronararterie fließt, das heißt der Anfangspunkt des Blutgefäßsegments, als erster Rahmen eines Bildes, und Aufnehmen des Koronar-Angiogrammbildes, wenn das Kontrastmittel zum Endpunkt des Blutgefäßsegments fließt, als ein N. Rahmen eines Bildes;

Lösen der Zeitdifferenz und Mittellinienlängendifferenz des N. Rahmens eines Bildes und eines (N-1). Rahmens, ..., eines (N-b). Rahmens, ..., eines (N-a). Rahmens, ..., des ersten Rahmens eines Bildes, nacheinander, wobei die Zeitdifferenzen jeweils $\Delta t_1$,..., $\Delta t_b$,..., $\Delta t_a$,..., $\Delta t_{N-1}$ sind; wobei die Mittellinienlängendifferenzen jeweils $\Delta L_1$,..., $\Delta L_b$,..., $\Delta L_a$,..., $\Delta L_{N-1}$ sind;

gemäß $v = \Delta L / \Delta t$, Erhalten der Blutströmungsgeschwindigkeit von dem N. Rahmen eines Bildes jeweils zu dem (N-1). Rahmen, ..., dem (N-b). Rahmen, ..., dem (N-a). Rahmen, ..., dem ersten Rahmen eines Bildes, wobei v die Blutströmungsgeschwindigkeit darstellt, wobei die Blutströmungsgeschwindigkeit jeweils $v_1$,..., $v_b$,..., $v_a$,..., $v_{N-1}$ ist.

11. Verfahren zum Berechnen einer angepassten Blutströmungsgeschwindigkeit im maximalen Hyperämiezustand basierend auf einem Mikrozirkulationswiderstandsindex nach Anspruch 7, **dadurch gekennzeichnet, dass** eine Art des Bestimmens einer Zeitdifferenz,

die für ein Kontrastmittel genommen wird, das durch das Blutgefäßsegment fließt, in zwei beliebigen Rahmen der zweidimensionalen Koronararterien-Angiogrammbilder mit der Differenz von $\Delta t$, und des Bestimmens einer Differenz der Mittellinienlänge eines Untersegments des Blutgefäßsegments, durch das das Kontrastmittel fließt, in den beiden Rahmen des zweidimensionalen Koronararterien-Angiogrammbildes mit der Differenz von $\Delta L$ und das Lösen der Blutströmungsgeschwindigkeit gemäß dem Verhältnis von $\Delta L$ zu $\Delta t$ umfasst:

Lösen der Zeitdifferenz und Mittellinienlängendifferenz des N. Rahmens und b. Rahmens, des (N-1). Rahmens und (b-1). Rahmens, ..., des (N-b-a). Rahmens und (N-a). Rahmens, ..., des (N-b+1). Rahmens und ersten Rahmens eines Bildes nacheinander;

gemäß $v = \Delta L / \Delta t$, Erlangen der Blutströ-

mungsgeschwindigkeit jeweils von dem N. Rahmen zu dem b. Rahmen, von dem (N-1). Rahmen zu dem (b-1). Rahmen, ..., von dem (N-b-a). Rahmen zu dem (N-a). Rahmen, ..., von dem (N-b+1). Rahmen zu dem ersten Rahmen eines Bildes, wobei v die Blutströmungsgeschwindigkeit darstellt.

12. Verfahren zum Berechnen einer angepassten Blutströmungsgeschwindigkeit im maximalen Hyperämiezustand basierend auf einem Mikrozirkulationswiderstandsindex nach Anspruch 7, **dadurch gekennzeichnet, dass** es nach der Art des Extrahierens einer Mittellinie des Blutgefäßsegments und vor der Art des Bestimmens einer Zeitdifferenz, die für ein Kontrastmittel genommen wird, das durch das Blutgefäßsegment fließt, in zwei beliebigen Rahmen der zweidimensionalen Koronararterien-Angiogrammbilder mit der Differenz von $\Delta t$, und des Bestimmens einer Differenz der Mittellinienlänge eines Untersegments des Blutgefäßsegments, durch das das Kontrastmittel fließt, in den beiden Rahmen des zweidimensionalen Koronararterien-Angiogrammbildes mit der Differenz von $\Delta L$, ferner umfasst:

Auslesen einer Gruppe von zweidimensionalen Koronararterien-Angiogrammbildern von mindestens zwei Körperpositionen;

Erhalten gemometrischer Strukturinformationen des Blutgefäßsegments;

Durchführen einer graphischen Verarbeitung an dem Blutgefäßsegment von Interesse;

Extrahieren einer Blutgefäß-Konturlinie des Blutgefäßsegments;

gemäß der geometrischen Strukturinformation des Blutgefäßsegments, Synthetisieren eines dreidimensionalen Blutgefäßmodells durch Projizieren der zweidimensionalen Koronoararterien-Angiogrammbilder der mindestens zwei Körperpositionen, die mittellinig und konturlinig des Blutgefäßes extrahiert wurden, auf eine dreidimensionale Ebene.

13. Verfahren zum Berechnen einer angepassten Blutströmungsgeschwindigkeit im maximalen Hyperämiezustand basierend auf einem Mikrozirkulationswiderstandsindex nach Anspruch 12, **dadurch gekennzeichnet, dass** eine Art des Lösens der Blutströmungsgeschwindigkeit gemäß dem Verhältnis von $\Delta L$ zu $\Delta t$ umfasst:

gemäß dem dreidimensionalen Blutgefäßmodell, Erhalten einer Mittellinie des dreidimensionalen Blutgefäßmodells, Korrigieren der Mittellinie, die aus den zweidimensionalen Koronar-Angiogrammbildern extrahiert wurde, und Korrigieren der Mittelliniendifferenz $\Delta L$, um $\Delta L'$ zu erlangen;

Lösen der Blutströmungsgeschwindigkeit v gemäß dem Verhältnis von $\Delta L'$ zu $\Delta t$.

14. Verfahren zum Erhalten eines Koronararterien-Blutgefäßauswertungsparameters basierend auf einem physiologischen Parameter, **dadurch gekennzeichnet, dass** es umfasst: das Verfahren zum Berechnen einer angepassten Blutströmungsgeschwindigkeit im maximalen Hyperämiezustand basierend auf einem Mikrozirkulationswiderstandsindex nach Anspruch 13.

15. Vorrichtung zum Berechnen einer angepassten Blutströmungsgeschwindigkeit im maximalen Hyperämiezustand basierend auf einem Mikrozirkulationswiderstandsindex, die ausgestaltet ist, das Verfahren zum Berechnen einer angepassten Blutströmungsgeschwindigkeit im maximalen Hyperämiezustand basierend auf einem Mikrozirkulationswiderstandsindex nach einem der Ansprüche 1 bis 13 auszuführen, **dadurch gekennzeichnet, dass** sie umfasst: eine Blutströmungsgeschwindigkeits-Erhalteinheit (1), eine Aortendruckwellenform-Erhalteinheit (2), eine Erhalteinheit von physiologischen Parametern (3), eine Einheit eines Mikrozirkulationswiderstandsindexes während der diastolischen Phase (4) und eine Anpassungsparametereinheit (5); wobei die Einheit eines Mikrozirkulationswiderstandsindexes während der diastolischen Phase (4) mit der Blutströmungsgeschwindigkeits-Erhalteinheit (1), der Aortendruckwellenform-Erhalteinheit (2) und der Erhalteinheit von physiologischen Parametern (3) verbunden ist;

wobei die Blutströmungsgeschwindigkeits-Erhalteinheit (1) dazu ausgestaltet ist, eine Blutströmungsgeschwindigkeit v zu erhalten; wobei die Aortendruckwellenform-Erhalteinheit (2) dazu ausgestaltet ist, in Echtzeit eine sich über die Zeit verändernde Aortendruckwellenform zu erhalten; wobei die Erhalteinheit von physiologischen Parametern (3) dazu ausgestaltet ist, physiologische Parameter eines Patienten zu erhalten, die Geschlecht und Krankheitsverlauf umfassen; wobei die Einheit eines Mikrozirkulationswiderstandsindexes während der diastolischen Phase (4) dazu ausgestaltet ist, die Blutströmungsgeschwindigkeit v, die Aortendruckwellenform und die physiologischen Parameter zu empfangen, die von der Blutströmungsgeschwindigkeits-Erhalteinheit (1), der Aortendruckwellenform-Erhalteinheit (2) und der Erhalteinheit von physiologischen Parametern (3) gesendet werden, und dann einen Mikrozirkulationswiderstandsindex iFMR während einer diastolischen Phase gemäß der Blutströmungsgeschwindigkeit v, der Aortendruckwellenform und den physiologischen Parametern zu erlangen;

wobei die Anpassungsparametereinheit (5) ausgestaltet ist, den iFMR-Wert der Einheit eines Mikrozirkulationswiderstandsindexes während der diastolischen Phase (4) zu empfangen; zu machen, dass ein Anpassungsparameter r gleich 1 ist, wenn der Mikrozirkulationswiderstandsindex während der diastolischen Phase iFMR<K ist; zu machen, dass der Anpassungsparameter r eine Formel

$$r = 1 - \frac{\text{iFMR} - K}{100}$$

erfüllt, wenn der Mikrozirkulationswiderstandsindex während der diastolischen Phase iFMR≥K ist; wobei K eine positive Zahl kleiner als 100 ist.

16. Vorrichtung zum Berechnen einer angepassten Blutströmungsgeschwindigkeit im maximalen Hyperämiezustand basierend auf einem Mikrozirkulationswiderstandsindex nach Anspruch 15, **dadurch gekennzeichnet, dass** sie ferner umfasst: eine Bildausleseeinheit (6), eine Blutgefäßsegmentextraktionseinheit (7) und eine Mittellinien-Extraktionseinheit (8), die in Reihe geschaltet sind, eine Zeitdifferenzeinheit (9) und die Erhalteinheit von physiologischen Parametern (3), die mit der Bildausleseeinheit (6) verbunden sind, wobei die Blutströmungsgeschwindigkeits-Erhalteinheit (1) jeweils mit der Zeitdifferenzeinheit (9) und einer Mittelliniendifferenzeinheit (10) verbunden ist; wobei die Mittelliniendifferenzeinheit (10) mit der Mittellinien-Extraktionseinheit (8) verbunden ist;

wobei die Bildausleseeinheit (6) dazu ausgestaltet ist, eine Gruppe von zweidimensionalen Koronararterien-Angiogrammbildern von mindestens einer Körperposition auszulesen; wobei die Blutgefäßsegmentextraktionseinheit (7) dazu ausgestaltet ist, zweidimensionale Koronararterien-Angiogrammbilder zu empfangen, die von der Bildausleseeinheit (6) gesendet werden, und ein Blutgefäßsegment von Interesse in den Bildern zu extrahieren; wobei die Mittellinien-Extraktionseinheit (8) dazu ausgestaltet ist, das Blutgefäßsegment zu empfangen, das von der Blutgefäßsegmentextraktionseinheit (7) gesendet wird, und die Mittellinie des Blutgefäßsegments zu extrahieren; wobei die Zeitdifferenzeinheit (9) ausgestaltet ist, zwei beliebige Rahmen der zweidimensionalen Koronararterien-Angiogrammbilder zu empfangen, die von der Bildausleseeinheit (6) gesendet werden, und eine Zeitdifferenz, die für ein Kontrastmittel genommen wird, das durch das Blutgefäßsegment fließt, in den beiden Rahmen des zweidimensionalen Koronararterien-Angiogrammbildes zu bestimmen, wobei die

Differenz $\Delta t$ ist;

wobei die Mittelliniendifferenzeinheit (10) dazu ausgestaltet ist, die Mittellinie eines Untersegments des Blutgefäßsegments, durch welches das Kontrastmittel fließt, in den beiden Rahmen des zweidimensionalen Koronararterien-Angiogrammbildes zu empfangen, das von der Mittellinien-Extraktionseinheit gesendet wird, und eine Differenz der Mittellinienlänge des Untersegments des Blutgefäßsegments, durch welches das Kontrastmittel fließt, in den beiden Rahmen des zweidimensionalen Koronararterien-Angiogrammbildes zu bestimmen, wobei die Differenz $\Delta L$ ist;

die Blutströmungsgeschwindigkeits-Erhalteinheit (1), die ein Blutströmungsgeschwindigkeits-Berechnungsmodul (101) und ein Berechnungsmodul für eine diastolische Blutströmungsgeschwindigkeit (102) umfasst, wobei das Blutströmungsgeschwindigkeits-Berechnungsmodul (101) jeweils mit der Zeitdifferenzeinheit (9) und der Mittelliniendifferenzeinheit (10) verbunden ist, wobei das Berechnungsmodul für eine diastolische Blutströmungsgeschwindigkeit (102) mit dem Blutströmungsgeschwindigkeits-Berechnungsmodul (101) verbunden ist;

wobei das Blutströmungsgeschwindigkeits-Berechnungsmodul (101) dazu ausgestaltet ist, $\Delta L$ und $\Delta t$ zu empfangen, die von der Zeitdifferenzeinheit (9) und der Mittelliniendifferenzeinheit (10) gesendet werden, und die Blutströmungsgeschwindigkeit gemäß dem Verhältnis von $\Delta L$ zu $\Delta t$ zu lösen;

wobei das Berechnungsmodul für eine diastolische Blutströmungsgeschwindigkeit (102) dazu ausgestaltet ist, die Blutströmungsgeschwindigkeit zu empfangen, die von dem Blutströmungsgeschwindigkeits-Berechnungsmodul (101) gesendet wird, und einen Maximalwert der Blutströmungsgeschwindigkeit als eine Blutströmungsgeschwindigkeit während einer diastolischen Phase auszuwählen;

wobei die Erhalteinheit von physiologischen Parametern (3) dazu ausgestaltet ist, die zweidimensionalen Koronararterien-Angiogrammbilder der Bildausleseeinheit (6) zu empfangen, einen physiologischen Parameter eines Patienten und Bildaufnahmewinkel zu erhalten und den physiologischen Parameter und die Bildaufnahmewinkel an die Mikrozirkulationswiderstandsindexeinheit während der diastolischen Phase (4) zu senden.

17. Vorrichtung zum Berechnen einer angepassten Blutströmungsgeschwindigkeit im maximalen Hyperämiezustand basierend auf einem Mikrozirkulationswiderstandsindex nach Anspruch 16, ferner umfassend: eine Blutgefäßskelett-Extraktionseinheit (11) und eine dreidimensionale Blutgefäß-Rekonstruktionseinheit (12), die beide mit der Bildausleseeinheit (6) verbunden sind, eine Konturlinien-Extraktionseinheit (13), die mit der Blutgefäßskelett-Extraktionseinheit (11) verbunden ist, wobei die dreidimensionale Blutgefäß-Rekonstruktionseinheit (12) mit der Erhalteinheit von physiologischen Parametern (3), der Mittellinien-Extraktionseinheit (8) und der Konturlinien-Extraktionseinheit (13) verbunden ist;

wobei die Blutgefäßskelett-Extraktionseinheit (11) dazu ausgestaltet ist, die zweidimensionalen Koronararterien-Angiogrammbilder zu empfangen, die von der Bildausleseeinheit (6) gesendet werden, und ein Blutgefäßskelett in den Bildern zu extrahieren;

wobei die Konturlinien-Extraktionseinheit (13) dazu ausgestaltet ist, das Blutgefäßskelett der Blutgefäßskelett-Extraktionseinheit (11) zu empfangen und eine Konturlinie des Blutgefäßsegments von Interesse gemäß dem Blutgefäßskelett zu extrahieren;

wobei die dreidimensionale Blutgefäß-Rekonstruktionseinheit (12) dazu ausgestaltet ist, die Konturlinie, die Bildaufnahmewinkel und die Mittellinie zu empfangen, die von der Konturlinien-Extraktionseinheit (13), der Erhalteinheit von physiologischen Parametern (3) und der Mittellinien-Extraktionseinheit (8) gesendet werden, und die zweidimensionalen Koronararterien-Angiogrammbilder zu empfangen, die von der Bildausleseeinheit (6) gesendet werden, um ein dreidimensionales Blutgefäßmodell durch Projizieren der zweidimensionalen Koronararterien-Angiogrammbilder von mindestens zwei Körperpositionen mit extrahierter Mittellinie und Konturlinie des Blutgefäßes auf eine dreidimensionale Ebene und gemäß der geometrischen Strukturinformation des Blutgefäßsegments zu synthetisieren;

wobei die Mittellinien-Extraktionseinheit (8) dazu ausgestaltet ist, die Mittellinie des Blutgefäßsegments aus dem dreidimensionalen Blutgefäßmodell der dreidimensionalen Blutgefäß-Rekonstruktionseinheit (12) erneut zu extrahieren und die Länge der Mittellinie erneut zu erhalten.

18. Koronararterien-Analysesystem, **dadurch gekennzeichnet, dass** es umfasst: die Vorrichtung zum Berechnen einer angepassten Blutströmungsgeschwindigkeit im maximalen Hyperämiezustand basierend auf einem Mikrozirkulationswiderstandsindex nach einem der Ansprüche 15 bis 17.

19. Computerspeichermedium, auf dem ein Computerprogramm gespeichert ist, das von einem Prozessor auszuführen ist, **dadurch gekennzeichnet, dass** das Verfahren zum Berechnen einer angepassten Blutströmungsgeschwindigkeit im maximalen Hyperämiezustand basierend auf einem Mikrozirkulationswiderstandsindex nach einem der Ansprüche 1 bis 13 durchgeführt wird, wenn das Computerprogramm durch den Prozessor ausgeführt wird.

**Revendications**

1. Procédé mis en œuvre par ordinateur pour calculer une vitesse de flux sanguin ajustée dans un état d'hyperémie maximale sur la base d'un indice de résistance microcirculatoire, **caractérisé en ce qu'**il comprend :

   l'acquisition d'un indice de résistance microcirculatoire iFMR pendant une phase diastolique en fonction d'une vitesse de flux sanguin v, d'une forme d'onde de pression aortique et d'un paramètre physiologique ;
   l'adaptation d'un paramètre d'ajustement r pour le rendre égal à 1 si l'indice de résistance microcirculatoire iFMR pendant la phase diastolique est inférieur à K ;
   l'adaptation du paramètre d'ajustement r pour qu'il satisfasse à la formule $r = 1 - \frac{iFMR - K}{100}$ si l'indice de résistance microcirculatoire iFMR pendant la phase diastolique est supérieur ou égal à K, dans lequel K est un nombre entier positif inférieur à 100 ;
   l'acquisition d'une vitesse de flux sanguin corrigée dans un état d'hyperémie maximale conformément à une formule $v' = rv_h$ ;
   dans lequel $v'$ représente la vitesse de flux sanguin corrigée dans l'état d'hyperémie maximale, et $v_h$ représente une vitesse de flux sanguin dans l'état d'hyperémie maximale.

2. Procédé de calcul d'une vitesse de flux sanguin ajustée dans un état d'hyperémie maximale sur la base d'un indice de résistance microcirculatoire selon la revendication 1, **caractérisé en ce que**,

$$\mathrm{v}_h = z v + x$$

   dans lequel $v_h$ représente la vitesse de flux sanguin dans l'état d'hyperémie maximale, $\bar{v}$ représente une vitesse de flux sanguin moyenne dans une zone de cycle cardiaque, z est une constante dans la plage de 1 à 3, et x est une constante dans la plage de 50 à 300 ; K=50.

3. Procédé de calcul d'une vitesse de flux sanguin ajustée dans un état d'hyperémie maximale sur la base d'un indice de résistance microcirculatoire selon la revendication 1, **caractérisé en ce que**, une manière d'acquérir un indice de résistance microcirculatoire iFMR pendant une phase diastolique en fonction d'une vitesse de flux sanguin v, d'une forme d'onde de pression aortique, et d'un paramètre physiologique comprend :

   la sélection d'une valeur maximale de la vitesse de flux sanguin v, c'est-à-dire, une vitesse de flux sanguin maximale $v_{max}$ pendant la phase diastolique ;
   une période correspondant à la $v_{max}$ étant la phase diastolique, l'acquisition d'une pression aortique moyenne pendant la phase diastolique selon la forme d'onde de pression aortique ;

$$\mathrm{iFMR} = \bar{P_a}/v_{max} \times k + c \; ;$$

$$\bar{P_a} = \frac{\sum_1^j (P_{a1} + P_{a2} \ldots P_{aj})}{j} \; ;$$

   dans lequel, $\bar{P_a}$ représente la pression aortique moyenne pendant la phase diastolique ; $P_{a1}$, $P_{a2}$, et $P_{aj}$ représentent des pressions aortiques correspondant à un premier point, un deuxième point, et un jième point dans la phase diastolique sur la forme d'onde de pression aortique, respectivement, et j représente le nombre de points de pression contenus dans la forme d'onde de pression aortique pendant la phase diastolique, $v_h$ représente la vitesse de flux sanguin dans l'état d'hyperémie maximale obtenue en sélectionnant une valeur maximale parmi toutes les vitesses de flux sanguin v ; k et c représentent les paramètres d'influence k=1~3, c=0~10.

4. Procédé de calcul d'une vitesse de flux sanguin ajustée dans un état d'hyperémie maximale sur la base d'un indice de résistance microcirculatoire selon la revendication 3, **caractérisé en ce que**, le paramètre d'influence k=axb, dans lequel a représente une valeur caractéristique de diabète, b représente une valeur caractéristique d'hypertension, et c représente un sexe.

5. Procédé de calcul d'une vitesse de flux sanguin ajustée dans un état d'hyperémie maximale sur la base d'un indice de résistance microcirculatoire selon la revendication 4, **caractérisé en ce que**, si un patient ne souffre pas de diabète, alors 0,5≤a≤1 ; si le patient souffre de diabète, alors 1<a≤2 ;

   si la pression artérielle du patient est supérieure ou égale à 90 mmHg, alors 1<b≤1,5 ; si la pression artérielle du patient est inférieure à 90 mmHg, alors 0,5≤b≤1 ;

si le patient est un homme, alors c=0 ; si le patient est une femme, alors c=3~10.

6. Procédé de calcul d'une vitesse de flux sanguin ajustée dans un état d'hyperémie maximale sur la base d'un indice de résistance microcirculatoire selon la revendication 5, **caractérisé en ce que**, si le patient ne souffre pas de diabète, alors a=1 ; si le patient souffre de diabète, alors a=2 ;

si la pression artérielle du patient est supérieure ou égale à 90 mmHg, alors b=1,5 ; si la pression artérielle du patient est inférieure à 90 mmHg, alors b=1 ;
si le patient est un homme, c=0 ; si le patient est une femme, c=5.

7. Procédé de calcul d'une vitesse de flux sanguin ajustée dans un état d'hyperémie maximale sur la base d'un indice de résistance microcirculatoire selon la revendication 1, **caractérisé en ce que**, une manière d'acquérir une vitesse de flux sanguin comprend :

la lecture d'un groupe d'images bidimensionnelles de coronarographie d'au moins une position du corps ;
l'extraction d'un segment de vaisseau sanguin d'intérêt du groupe d'images bidimensionnelles de coronarographie ;
l'extraction d'une ligne médiane du segment de vaisseau sanguin ;
la détermination d'une différence de temps nécessaire à un agent de contraste circulant à travers le segment de vaisseau sanguin dans deux trames quelconques des images bidimensionnelles de coronarographie, la différence étant $\Delta t$, et la détermination d'une différence de longueur de ligne médiane d'un sous-segment du segment de vaisseau sanguin à travers lequel circule l'agent de contraste dans les deux trames de l'image bidimensionnelle de coronarographie, la différence étant $\Delta L$ ;
la résolution de la vitesse de flux sanguin selon un rapport de $\Delta L$ à $\Delta t$.

8. Procédé de calcul d'une vitesse de flux sanguin ajustée dans un état d'hyperémie maximale sur la base d'un indice de résistance microcirculatoire selon la revendication 7, **caractérisé en ce que**, une manière d'extraire un segment de vaisseau sanguin d'intérêt du groupe d'images bidimensionnelles de coronarographie comprend :

la sélection de N trames des images bidimensionnelles de coronarographie du groupe d'images bidimensionnelles de coronarographie ;
l'acquisition du segment de vaisseau sanguin d'intérêt en choisissant un point de départ et un point de fin du vaisseau sanguin d'intérêt sur les images bidimensionnelles de coronarographie.

9. Procédé de calcul d'une vitesse de flux sanguin ajustée dans un état d'hyperémie maximale sur la base d'un indice de résistance microcirculatoire selon la revendication 7, **caractérisé en ce que**, une manière d'extraire la ligne médiane du segment de vaisseau sanguin comprend :

l'extraction d'un squelette de vaisseau sanguin des images bidimensionnelles de coronarographie ;
en fonction d'une direction d'extension du segment de vaisseau sanguin et d'un principe d'obtention du chemin le plus court entre deux points ;
l'extraction de la ligne médiane du segment de vaisseau sanguin le long du squelette de vaisseau sanguin.

10. Procédé de calcul d'une vitesse de flux sanguin ajustée dans un état d'hyperémie maximale sur la base d'un indice de résistance microcirculatoire selon la revendication 7, **caractérisé en ce que**, une manière de déterminer une différence de temps nécessaire à un agent de contraste circulant à travers le segment de vaisseau sanguin dans deux trames quelconques des images bidimensionnelles de coronarographie, la différence étant $\Delta t$, et de déterminer une différence de longueur de ligne médiane d'un sous-segment du segment de vaisseau sanguin à travers lequel circule l'agent de contraste dans les deux trames de l'image bidimensionnelle de coronarographie, la différence étant $\Delta L$, et de résoudre la vitesse de flux sanguin selon le rapport de $\Delta L$ à $\Delta t$ comprend :

la capture de l'image de coronarographie lorsque l'agent de contraste circule vers l'entrée de l'artère coronaire, c'est-à-dire, le point de départ du segment de vaisseau sanguin en tant que première trame d'image, et la capture de l'image de coronarographie lorsque l'agent de contraste circule vers le point de fin du segment de vaisseau sanguin en tant que Nième trame d'image ;
la résolution de la différence temporelle et de la différence de longueur de ligne médiane de la Nième trame d'image et d'une (N-1) ième trame d'image, ..., d'une (N-b) ième trame,..., d'une (N-a)ième trame, ..., de la première trame d'image, successivement, avec les différences temporelles étant $\Delta t_1$, ..., $\Delta t_b$, ..., $\Delta t_a$, ..., $\Delta t_{N-1}$, respectivement ; les différences de longueur de ligne médiane étant $\Delta L_1$, ..., $\Delta L_b$, ..., $\Delta L_a$, ...,

$\Delta L_{N-1}$, respectivement ;

selon $v = \Delta L / \Delta t$, l'obtention de la vitesse de flux sanguin de la Nième trame d'image à la (N-1) ième trame, ..., la (N-b) ième trame, ..., la (N-a) ième trame, ..., la première trame d'image, respectivement, dans lequel v représente la vitesse de flux sanguin, la vitesse de flux sanguin étant v$_1$, ..., v$_b$, ..., v$_a$, ..., v$_{N-1}$, respectivement.

11. Procédé de calcul d'une vitesse de flux sanguin ajustée dans un état d'hyperémie maximale sur la base d'un indice de résistance microcirculatoire selon la revendication 7, **caractérisé en ce que**, une manière de déterminer une différence de temps nécessaire à un agent de contraste circulant à travers le segment de vaisseau sanguin dans deux trames quelconques d'images bidimensionnelles de coronarographie, la différence étant $\Delta t$, et de déterminer d'une différence de longueur de ligne médiane d'un sous-segment du segment de vaisseau sanguin à travers lequel circule l'agent de contraste dans les deux trames de l'image bidimensionnelle de coronarographie, la différence étant $\Delta L$, et de résoudre la vitesse de flux sanguin selon le rapport de $\Delta L$ à $\Delta t$ comprend :

la résolution de la différence temporelle et de la différence de longueur de ligne médiane de la Nième trame et d'une bième trame, de la (N-1)ième trame et d'une (b-1)ième trame, ..., de la (N-b-a) ième trame à une (N-a) ième trame, ..., de la (N-b+1)ième trame et de la première trame d'image, successivement ;

selon $v = \Delta L / \Delta t$, l'obtention de la vitesse de flux sanguin à de la Nième trame à la bième trame, de la (N-1)ième trame à la (b-1) ième trame, ..., de la (N-b-a) ième trame à la (N-a) ième trame, ..., de la (N-b+1) ième trame à la première trame d'image, respectivement, dans lequel v représente la vitesse de flux sanguin.

12. Procédé de calcul d'une vitesse de flux sanguin ajustée dans un état d'hyperémie maximale sur la base d'un indice de résistance microcirculatoire selon la revendication 7, **caractérisé en ce que**, suite à la manière d'extraire une ligne médiane du segment de vaisseau sanguin, et avant la manière de déterminer une différence de temps nécessaire à un agent de contraste circulant à travers le segment de vaisseau sanguin dans deux trames quelconques d'images bidimensionnelles de coronarographie, la différence étant $\Delta t$, et de déterminer une différence de longueur de ligne médiane d'un sous-segment du segment de vaisseau sanguin à travers lequel circule l'agent de contraste dans les deux trames de l'image bidimensionnelle de coronarographie, la différence étant $\Delta L$, comprend en outre :

la lecture d'un groupe d'images bidimensionnelles de coronarographie d'au moins deux positions du corps ; l'acquisition d'informations de structure géométrique du segment de vaisseau sanguin ; la réalisation d'un traitement graphique sur le segment de vaisseau sanguin d'intérêt ; l'extraction d'une ligne de contour de vaisseau sanguin du segment de vaisseau sanguin ; en fonction des informations de structure géométrique du segment de vaisseau sanguin, la synthèse d'un modèle de vaisseau sanguin tridimensionnel en projetant les images bidimensionnelles de coronarographie des au moins deux positions du corps, dont ont été extraites la ligne médiane et la ligne de contour du vaisseau sanguin, sur un plan tridimensionnel.

13. Procédé de calcul d'une vitesse de flux sanguin ajustée dans un état d'hyperémie maximale sur la base d'un indice de résistance microcirculatoire selon la revendication 12, **caractérisé en ce que**, une manière de résoudre la vitesse de flux sanguin selon le rapport de $\Delta L$ à $\Delta t$ comprend :

en fonction du modèle de vaisseau sanguin tridimensionnel, l'acquisition d'une ligne médiane du modèle de vaisseau sanguin tridimensionnel, la correction de la ligne médiane extraite des images bidimensionnelles de coronarographie, et la correction de la différence de ligne médiane $\Delta L$ pour obtenir $\Delta L'$ ; la résolution de la vitesse de flux sanguin v selon le rapport de la $\Delta L'$ à la $\Delta t$.

14. Procédé d'acquisition d'un paramètre d'évaluation de vaisseau sanguin d'artère coronaire sur la base d'un paramètre physiologique, **caractérisé en ce qu'**il comprend : le procédé de calcul d'une vitesse de flux sanguin ajustée dans un état d'hyperhémie maximale sur la base d'un indice de résistance microcirculatoire selon la revendication 13.

15. Appareil pour calculer une vitesse de flux sanguin ajustée dans un état d'hyperhémie maximale sur la base d'un indice de résistance microcirculatoire, configuré pour mettre en oeuvre le procédé de calcul d'une vitesse de flux sanguin ajustée dans un état d'hyperhémie maximale sur la base d'un indice de résistance microcirculatoire selon l'une quelconque des revendications 1 à 13, **caractérisé en ce qu'**il comprend : une unité d'acquisition de vitesse de flux sanguin (1), une unité d'acquisition de forme d'onde de pression aortique (2), une unité d'acquisition de paramètres physiologiques (3), une unité d'indice de résistance microcirculatoire pendant la phase diastolique (4) et une unité de paramètre d'ajustement (5) ; l'unité d'indice de résistance microcirculatoire pendant la phase diastolique (4) étant connectée à l'unité d'acquisition de vitesse de flux sanguin (1), à l'unité d'acquisition de forme d'onde de pression aortique (2) et à l'unité d'acquisition de paramètres physiologiques (3) ;

l'unité d'acquisition de vitesse de flux sanguin (1) étant configurée pour acquérir une vitesse de flux sanguin v ;

l'unité d'acquisition de forme d'onde de pression aortique (2) étant configurée pour acquérir, en temps réel, une forme d'onde de pression aortique évoluant au fil du temps ;

l'unité d'acquisition de paramètres physiologiques (3) étant configurée pour acquérir des paramètres physiologiques d'un patient, comprenant le sexe et les antécédents médicaux ;

l'unité d'indice de résistance microcirculatoire pendant la phase diastolique (4) étant configurée pour recevoir la vitesse de flux sanguin v, la forme d'onde de pression aortique, et les paramètres physiologiques envoyés par l'unité d'acquisition de vitesse de flux sanguin (1), l'unité d'acquisition de forme d'onde de pression aortique (2), et l'unité d'acquisition de paramètres physiologiques (3), puis pour obtenir un indice de résistance microcirculatoire iFMR pendant une phase diastolique en fonction de la vitesse de flux sanguin v, de la forme d'onde de pression aortique, et des paramètres physiologiques ;

l'unité de paramètre d'ajustement (5) étant configurée pour recevoir une valeur iFMR de l'unité d'indice de résistance microcirculatoire pendant la phase diastolique (4) ; rendre un paramètre d'ajustement r égal à 1 si l'un indice de résistance microcirculatoire pendant la phase diastolique iFMR<K ; adapter le paramètre d'ajustement r pour satisfaire à la formule

$$r = 1 - \frac{iFMR - K}{100}$$

si l'indice de résistance microcirculatoire pendant la phase diastolique iFMR$\geq$K ; dans lequel K est un nombre entier positif inférieur à 100.

16. Appareil pour calculer une vitesse de flux sanguin ajustée dans un état d'hyperhémie maximale sur la base d'un indice de résistance microcirculatoire selon la revendication 15, **caractérisé en ce qu'**il comprend en outre : une unité de lecture d'images (6), une unité d'extraction de segment de vaisseau sanguin (7), et une unité d'extraction de ligne médiane (8) connectées en séquence, une unité de différence temporelle (9) et l'unité d'acquisition de paramètres physiologiques (3) étant connectées à l'unité de lecture d'images (6), l'unité d'acquisition de vitesse de flux sanguin (1) étant connectée à l'unité de différence temporelle (9) et à une unité de différence de ligne médiane (10), respectivement ; l'unité de différence de ligne médiane (10) étant connectée à l'unité d'extraction de ligne médiane (8) ;

l'unité de lecture d'images (6) étant configurée pour lire un groupe d'images bidimensionnelles de coronarographie d'au moins une position du corps ;

l'unité d'extraction de segment de vaisseau sanguin (7) étant configurée pour recevoir des images bidimensionnelles de coronarographie envoyées par l'unité de lecture d'images (6), et pour extraire un segment de vaisseau sanguin d'intérêt dans les images ;

l'unité d'extraction de ligne médiane (8) étant configurée pour recevoir le segment de vaisseau sanguin envoyé par l'unité d'extraction de segment de vaisseau sanguin (7), et pour extraire la ligne médiane du segment de vaisseau sanguin ;

l'unité de différence temporelle (9) étant configurée pour recevoir deux trames quelconques des images bidimensionnelles de coronarographie envoyées par de l'unité de lecture d'images (6) et pour déterminer une différence de temps nécessaire à un agent de contraste circulant à travers le segment de vaisseau sanguin dans les deux trames des images bidimensionnelles de coronarographie, la différence étant $\Delta t$ ;

l'unité de différence de ligne médiane (10) étant configurée pour recevoir la ligne médiane d'un sous-segment du segment de vaisseau sanguin parcouru par l'agent de contraste dans les deux trames de l'image bidimensionnelle de coronarographie envoyée par l'unité d'extraction de ligne médiane, et pour déterminer une différence de longueur de ligne médiane du sous-segment du segment de vaisseau sanguin à travers lequel circule l'agent de contraste dans les deux trames de l'image bidimensionnelle de coronarographie, la différence étant $\Delta L$ ;

l'unité d'acquisition de vitesse de flux sanguin (1), comprenant un module de calcul de vitesse de flux sanguin (101) et un module de calcul de vitesse de flux sanguin diastolique (102), le module de calcul de vitesse de flux sanguin (101) étant respectivement connecté à l'unité de différence temporelle (9) et à l'unité de différence de ligne médiane (10), le module de calcul de vitesse de flux sanguin diastolique (102) étant connecté au module de calcul de vitesse de flux sanguin (101) ;

le module de calcul de vitesse de flux sanguin (101) étant configuré pour recevoir la $\Delta L$ et la $\Delta t$ envoyées par l'unité de différence temporelle (9) et l'unité de différence de ligne médiane (10), et pour résoudre la vitesse de flux sanguin en fonction du rapport $\Delta L$ à $\Delta t$ ;

le module de calcul de vitesse de flux sanguin diastolique (102) étant configuré pour recevoir la vitesse de flux sanguin envoyée par le module de calcul de vitesse de flux sanguin (101), et pour sélectionner une valeur maximale de la vitesse de flux sanguin en tant que vitesse de flux sanguin pendant une phase diastolique ;

l'unité d'acquisition de paramètres physiologiques (3) étant configurée pour recevoir les images bidimensionnelles de coronarographie de l'unité de lecture d'images (6), pour acquérir un paramètre physiologique d'un patient et des angles de capture d'images, et pour transmettre le paramètre physiologique et les angles de capture d'images à l'unité d'indice de résistance microcirculatoire pendant la phase diastolique (4).

17. Appareil pour calculer une vitesse de flux sanguin ajustée dans un état d'hyperhémie maximale sur la base d'un indice de résistance microcirculatoire selon la revendication 16, comprenant en outre : une unité d'extraction de squelette de vaisseau sanguin (11) et une unité de reconstruction de vaisseau sanguin tridimensionnel (12), toutes deux connectées à l'unité de lecture d'images (6), une unité d'extraction de ligne de contour (13) étant connectée à l'unité d'extraction de squelette de vaisseau sanguin (11), l'unité de reconstruction de vaisseau sanguin tridimensionnel (12) étant connectée à l'unité d'acquisition de paramètres physiologiques (3), à l'unité d'extraction de ligne médiane (8) et à l'unité d'extraction de ligne de contour (13) ;

> l'unité d'extraction de squelette de vaisseau sanguin (11) étant configurée pour recevoir les images bidimensionnelles de coronarographie envoyées par l'unité de lecture d'images (6), et pour extraire un squelette de vaisseau sanguin dans les images ;
> l'unité d'extraction de ligne de contour (13) étant configurée pour recevoir le squelette de vaisseau sanguin de l'unité d'extraction de squelette de vaisseau sanguin (11), et pour extraire une ligne de contour du segment de vaisseau sanguin d'intérêt conformément au squelette de vaisseau sanguin ;
> l'unité de reconstruction de vaisseau sanguin tridimensionnel (12) étant configurée pour recevoir la ligne de contour, les angles de capture d'images et la ligne médiane envoyés par l'unité d'extraction de ligne de contour (13), l'unité d'acquisition de paramètres physiologiques (3) et l'unité d'extraction de ligne médiane (8), et pour recevoir les images bidimensionnelles de coronarographie envoyées par l'unité de lecture d'images (6) afin de synthétiser un modèle de vaisseau sanguin tridimensionnel en projetant les images bidimensionnelles de coronarographie d'au moins deux positions du corps avec la ligne médiane et la ligne de contour extraites du vaisseau sanguin sur un plan tridimensionnel et en fonction des informations de structure géométrique du segment de vaisseau sanguin ;

l'unité d'extraction de ligne médiane (8) étant configurée pour réextraire la ligne médiane du segment de vaisseau sanguin du modèle de vaisseau sanguin tridimensionnel de l'unité de reconstruction de vaisseau sanguin tridimensionnel (12), et pour réacquérir la longueur de la ligne médiane.

18. Système d'analyse d'artère coronaire, **caractérisé en ce qu'**il comprend : l'appareil pour calculer une vitesse de flux sanguin ajustée dans un état d'hyperhémie maximale sur la base d'un indice de résistance microcirculatoire selon l'une quelconque des revendications 15 à 17.

19. Support de stockage informatique présentant, stocké sur celui-ci, un programme informatique à exécuter par un processeur, **caractérisé en ce que**, le procédé de calcul d'une vitesse de flux sanguin ajustée dans un état d'hyperhémie maximale sur la base d'un indice de résistance microcirculatoire selon l'une quelconque des revendications 1 à 13 est mis en oeuvre lorsque le programme informatique est exécuté par le processeur.

S100 — acquiring an index for microcirculatory resistance iFMR during a diastolic phase according to a blood flow velocity v, an aortic pressure waveform, and an physiological parameter

S200 — Making an adjustment parameter r equal to 1 if the index for microcirculatory resistance iFMR during the diastolic phase is less than K; making the adjustment parameter r satisfy a formula $r = 1 - (iFMR - K)/100$ if the index for microcirculatory resistance iFMR during the diastolic phase is greater than or equal to K;

S300 — acquiring a corrected blood flow velocity in a maximum hyperemia state according to a formula $v' = rv_h$.

FIG.1

S110 — reading a group of two-dimensional coronary artery angiogram images of at least one body position;

S120 — extracting a blood vessel segment of interest from the group of two-dimensional coronary artery angiogram images;

S130 — extracting a centerline of the blood vessel segment;

S140 — determining a difference in time taken for a contrast agent flowing through the blood vessel segment in any two frames of the two-dimensional coronary artery angiogram images, and determining a difference in centerline length of a sub-segment of the blood vessel segment through which the contrast agent flows in the two frames of two-dimensional coronary artery angiogram image;

S150 — solving the blood flow velocity v according to a ratio of $\Delta L$ to $\Delta t$.

S160 — selecting a maximum value of the blood flow velocity v, i.e., a maximum blood flow velocity $v_{max}$ during the diastolic phase;

S170 — a time period corresponding to the $v_{max}$ being the diastolic phase, acquiring an average aortic pressure during the diastolic phase according to the aortic pressure waveform;

S180 — obtaining a iFMR value according to the calculation formula: $iFMR = \overline{Pa}/v_{max} \times k + c$;

FIG.2

S121 — selecting N frames of the two-dimensional coronary artery angiogram images from the group of two-dimensional coronary artery angiogram images;

S122 — acquiring the blood vessel segment of interest by picking a beginning point and an ending point of the blood vessel of interest on the two-dimensional coronary artery angiogram images;

FIG.3

S131 — extracting a blood vessel skeleton from the two-dimensional coronary artery angiogram images;

S132 — according to the extension direction of the blood vessel segment and the principle of obtaining the shortest path between two points;

S133 — extracting the centerline of the blood vessel segment along the blood vessel skeleton.

FIG.4

S141I

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;

S142I

solving the time difference and centerline length difference of the N-th frame of image and a (N-1)th frame, ..., a (N-b)th frame, ..., a (N-a)th frame, ..., the first frame of image, successively, with the time differences being $\Delta t_1, ..., \Delta t_b, ..., \Delta t_a, ..., \Delta t_{N-1}$, respectively, the centerline length differences being $\Delta L_1, ..., \Delta L_b, ..., \Delta L_a, ..., \Delta L_{N-1}$, respectively;

S143I

according to $v = \Delta L / \Delta t$, obtaining the blood flow velocity from the N-th frame of image to the (N-1)th frame, ..., the (N-b)th frame, ..., the (N-a)th frame, ..., the first frame of image, respectively, wherein v represents the blood flow velocity, with the blood flow velocity being $v_1, ..., v_b, ..., v_a, ..., v_{N-1}$, respectively.

FIG.5

S141II

taking the coronary angiogram image when the contrast agent flows to the inlet of the coronary artery, that is, the beginning point of the blood vessel segment as a first frame of image, and taking the coronary angiogram image when the contrast agent flows to the ending point of the blood vessel segment as a N-th frame of image;

S142II

solving the time difference and centerline length difference of the N-th frame and b-th frame, of the (N-1)th frame and (b-1)th frame, ..., of the (N-b)th frame and (N-a)th frame, ..., of the (N-b+1)th frame and first frame of image, successively;

S143II

according to $v = \Delta L / \Delta t$, obtaining the blood flow velocity from the N-th frame to the b-th frame, from the (N-1)th frame to the (b-1)th frame, ..., from the (N-b-a)th frame to the (N-a)th frame, ..., from the (N-b+1)th frame to the first frame of image, respectively, wherein v represents the blood flow velocity.

FIG.6

adjustment
parameter unit 5

physiological parameter
acquisition unit 3

unit of index for
microcirculatory
resistance during
diastolic phase 4

aortic pressure
waveform
acquisition unit 2

blood flow velocity
acquisition unit 1

FIG.7

unit of flow velocity
in maximum
hyperemia state 15

contour line
extraction unit 13

blood vessel skeleton
extraction unit 11

adjustment parameter
unit 5

flow velocity
correction unit 14

three-dimensional
blood vessel
reconstruction unit 12

image reading
unit 6

physiological
parameter acquisition
unit 3

unit of index for
microcirculatory
resistance during
diastolic phase 4

aortic pressure
waveform acquisition
unit 2

blood vessel segment
extraction unit 7

time difference
unit 9

diastolic blood flow
velocity calculation
module 102

centerline extraction
unit 8

centerline difference
unit 10

blood flow velocity
calculation module
101

blood flow velocity
acquisition unit 1

FIG.8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110384494 A

- CN 108550189 A